# EUROPEAN PATENT APPLICATION

(11) **EP 4 293 042 A1**
(43) Date of publication of application: **20.12.2023**
(21) Application number: 21925175.8
(22) Date of filing: 09.02.2021
(51) Int. Cl.: C07K 14/725, C12N 15/12, C12N 15/63, C12N 5/10, A61K 38/17, A61P 35/00

(54) **TCR AND APPLICATION THEREOF**

(71) Applicant: Liyang TCR Biotherapeutics Co., Ltd, Liyang, Jiangsu 213300 (CN)
(72) Inventor: WU, Xianhui, Shenzhen, Guangdong 518000 (CN)
(74) Representative: dompatent von Kreisler Selting Werner - Partnerschaft von Patent- und Rechtsanwälten mbB
(86) International application number: PCT/CN2021/076342
(87) International publication number: WO 2022/170509

(57) **Abstract**

Disclosed in the present invention are a TCR and an application thereof. The TCR comprises a TCR α-chain variable region and/or a TCR β-chain variable region. The TCR can specifically recognize and bind to HLA-A*02:01/SLLMWITQC, and is characterized in that the affinity K_{D1} of the TCR for the HLA-A*02:01/SLLMWITQC is 0.1-10 µM. The TCR can also specifically recognize and bind to one or more of HLA-A*02:03/SLLMWITQC, HLA-A*02:09/SLLMWITQC, HLA-A*02:12/SLLMWITQC, and HLA-A*02:16/SLLMWITQC, and the affinity K_{D2} is 0.1-85 µM. The TCR in the present invention has extremely high affinity, excellent safety, and wider applicability.

## Description

### TECHNICAL FIELD

The present invention belongs to the field of tumor therapy and molecular immunology, and in particular relates to a TCR and an application thereof.

### BACKGROUND

NY-ESO-1 belongs to the Cancer-Testis Antigen (CTA) family, which is not expressed in normal tissues, but highly expressed in testis, ovarian tissues, neuroblastoma (Cancer Res, 2003, 63, 6948), sarcoma (Int J Cancer, 2001, 94, 252), malignant melanoma (Clin Cancer Res, 2006, 12, 764) and many other types of tumors *(*PNAS 1997, 94, 1914; Int J Cancer 2001, 92, 856; Cancer Res. 2003, 63, 6076), and has relatively high expression in prostate cancer, bladder cancer, breast cancer, myeloid malignant leukemia, hepatocellular carcinoma, oral squamous carcinoma (Anticancer RES, 2009, 29, 5125), and esophageal cancer (C1 in Cancer Res, 2004, 10, 6551) as well.

The SLLMWITQC peptide of NY-ESO-1, presented on the cell surface via HLA-A*02, becomes an ideal target for TCR-T cell immunotherapy. The safety and initial effectiveness against solid tumors of this target has been validated in clinical studies and clinical trials (Nat Med. 2015, 21, 914; Clin Cancer Res. 2015, 21, 1019). Currently, several products of TCR-T immunotherapy are available for targeting HLA-A*02/SLLMWITQC. Some products have been reported, such as CN106632658A, CN106632660A, US2016159881A1, and CN108117596, etc. The effectiveness and long-term safety of several clinical trials have been reported as well (Cancer Discov, 2018, 8, 944; Blood Adv. 2019, 3, 2020). Compared with traditional chemotherapy, radiotherapy, and other therapies, this type of immunological product has the advantage that causes no damage to normal cells of the donor, making it an ideal target for TCR-T immune cell therapy.

The T cell receptor (TCR) targeting HLA-A*02:01/SLLMWITQC of cancer-testis self antigen, generally has an affinity *K_{D}* value weaker than 10 µM, which therefore lead to an ineffective killing of tumor cells that display this peptide, unless its affinity K_{D} is improved to 0.5/1.0 - 10 µM *(*PNAS, 2013. 110, 6973; Eur. J. Immunol. 2012, 42, 3174;), or even to 0.1 - 10 µM. While utilizing the affinity optimization *in vitro* to improve the affinity is prone to introduce several issues such as non-specificity and cross-reactivity, it is not easy to obtain mutations that meet the requirements of safety and efficacy. Meanwhile, benefiting from the TCRs obtained by *in vitro* affinity optimization method, the number of patients are limited due to the diversity of HLA haplotype. Thus, TCRs that target multiple HLA-A*02:0x haplotype (x may be, for example, 1, 3, 9, 12, 16, etc.) and satisfy the requirements of efficacy and safety, are urgently needed to expand the therapeutic effects and the number of beneficiaries.

### CONTENT OF THE PRESENT INVENTION

The technical problem to be solved in the present invention is to provide a TCR and an application thereof for overcoming the issue on limited number of TCR for recognizing and binding to HLA-A*02:01/SLLMWITQC, as well as being able to recognize other HLA-A*02:0x in the prior art. The TCR of the present invention has extremely high affinity and excellent safety, with an affinity *K_{D}* of 0.1-10 µM against HLA-A*02:01/SLLMWITQC; in addition, the TCR of the present invention is also able to bind to other complexes such as HLA-A*02:03/SLLMWITQC, HLA-A*02:09/SLLMWITQC, HLA-A*02.12/SLLMWITQC, and HLA-A*02:16/SLLMWITQC, and thus has wider applicability.

MHC molecules, the member of immunoglobulin superfamily, may be class I or class II MHC molecules, which are specific for antigen presentation. Different individuals possess different MHCs that may present different short peptides of a protein antigen to the surface of each respective antigen presenting cells (APCs). Human MHCs are commonly referred as HLA genes or HLA complexes.

T cell receptor (TCR), is the only receptor for the specific antigen presented on major histocompatibility complex (MHC). In immune system, direct physical contact between T cells and APCs is triggered by the binding of antigen-specific TCR to pMHC complex, then interaction occurs among other cell membrane surface molecules of both T cells and APCs, which causes a series of subsequent cell signaling and other physiological responses, thereby enabling T cells with different antigen specificities to exert immune effects on their target cells.

TCR, the functional unit of a T lymphocyte to recognize an antigen, belongs to the immunoglobulin superfamily, the coding chains of which include α, β, γ and δ four chains. TCR is a glycoprotein on the surface of cell membrane that exists in form of the heterodimer consisting of α chain/β chain or γ chain/δ chain. 95% TCRs in peripheral blood are heterodimers composed of two polypeptide chains, α and β. However, the recombinant TCR may also consist of a single TCR β chain or a TCR α chain, which has been proven to be able to bind the antigenic peptide-MHC molecules (WO 2005/113595).

Generally, α- and β- chains each contains a variable region, a joining region and a constant region. A short diversity region between variable region and joining region is usually included in β-chain, which is often considered as part of the joining region. Each variable region contains three CDRs (complementarity-determining regions) that embedded in framework regions, CDR1, CDR2 and CDR3. CDR regions determine the binding of TCR to pMHC complex, wherein CDR3 is formed by recombination of the variable region and the joining region, which is referred as the hypervariable region. The α and β chains of TCR are generally considered to have two "domains", *i.e.* variable domain and constant domain, wherein the variable domain is composed of ligated variable region and joining region. The sequence of TCR constant domain may be found in the public database of International Immunogenetics Information System (IMGT), for example, the sequence of the constant domain of α chains acts as TRAC (which may also be referred as TRAC*01), the sequence of the constant domain of β chains acts as TRBC1 (which may also be referred as TRBC1*01) or TRBC2 (which may also be referred as TRBC2*01). In addition, α and β chains of TCR also contain transmembrane regions and short cytoplasmic regions.

In addition, α chain is formed by the rearrangements of germline genes TRAV, TRAJ, and TRAC; β-chain is formed by the rearrangements of germline genes TRBV, TRBD, TRBJ, and TRBC. After the rearrangements of different V(D)Js, different numbers of nucleotides are randomly inserted during the joining of V-J (or V-D and D-J), forming a variable region (N) which act as the complementarity-determining region 3 (CDR3). Such random insertion endow the sequences of TCR α and β chains with high degree of diversity. In different clones of T lymphocytes, the lengths and nucleotide sequences of CDR3 are different during TCR rearrangements, wherein CDR3 is the region that TCR specifically recognizes the antigen, and determines the specificity of a TCR.

Furthermore: in addition to the mutations explicitly described in the present invention, there are conserved modifications, or conserved substitutions known in the art. The conserved modifications or the conserved substitutions refer to the substitution of amino acids in the protein by other amino acids with similar characteristics (e.g., charge, side chain size, hydrophobicity/hydrophilicity, backbone conformation, rigidity, etc.), which enables frequent changes without altering the biological activity of the protein. It is known to those skilled in the art that, in general, a single amino acid substitution in the non-essential region of a polypeptide do not substantially alter the biological activity (see e.g. Watson et al. (1987) Molecµlar Biology of the Gene, The Benjamin/Cummings Pub. Co., page 224, (4th edition)). In addition, substitutions of amino acids with similar structure or function are unlikely to destruct the biological activity. The common conserved substitutions of amino acids are as follows:

| Original Residue | Examplary Substituent | Preferred Substituent |
|---|---|---|
| Ala(A) | Val, Leu, Ile | Val |
| Arg(R) | Lys, Gln, Asn | Lys |
| Asn(N) | Gln, His, Asp, Lys, Arg | Gln |
| Asp(D) | Glu, Asn | Glu |
| Cys(C) | Ser, Ala | Ser |
| Gln(Q) | Asn, Glu | Asn |
| Glu(E) | Asp, Gln | Asp |
| Gly(G) | Ala | Ala |
| His(H) | Arg, Asn, Gln, Lys | Arg |
| Ile(I) | Leu, Val, Met, Ala, Phe, Ile | Leu |
| Leu(L) | Ile, Nle, Val, Met, Ala, Phe | Ile |
| Lys(K) | Arg, Gln, Asn | Arg |
| Met(M) | Leu, Phe, Ile | Aeu |
| Phe(F) | Tyr, Leu, Val, Ile, Ala | Tyr |
| Pro(P) | Ala | Ala |
| Ser(S) | Thr | Thr |
| Thr(T) | Ser | Ser |
| Trp(W) | Tyr, Phe | Tyr |
| Tyr(Y) | Phe, Trp, Thr, Ser | Phe |
| Val(V) | Leu, Ile, Met, Phe, Ala, Nle | Leu |

Through many experiments, the inventors find a novel wild-type (natural) TCR which may specifically recognize HLA-A*02:01/SLLMWITQC. Based on this T cell receptor, the inventors used protein engineering approaches to obtain a stably folded scTCR mutant sequence. Using this scTCR sequence, a series of heterodimeric TCR mutants with high affinity and safety were obtained by further phage displays, wherein the affinity *K_{D}* is 0.1-10 µM; moreover, such heterodimeric TCR mutants are surprisingly found to recognize other different HLA-A*02 targets, which have a wider applicability. Furthermore, these mutants exhibit specific killing activity against various tumor cell lines after transduction into CD8⁺ T cells via lentivirus.

The present invention addresses the above technical problems mainly by the following technical solutions.

The present invention provides a TCR comprising a TCR α-chain variable region and/or a TCR β-chain variable region, wherein the TCR can specifically recognize and bind to HLA-A*02:01/SLLMWITQC, the affinity *K_{D1}* value of the TCR binding to the HLA-A*02:01/SLLMWITQC is 0.1-10 µM; the TCR may also specifically recognize and bind to one or more of HLA-A*02:03/SLLMWITQC, HLA-A*02:09/SLLMWITQC, HLA-A*02:12/SLLMWITQC, and HLA-A*02:16/SLLMWITQC, and the affinity *K_{D2}* value is 0.1-85 µM, preferably 0.57-27 µM, more preferably 1.3-10 µM.

Herein: the affinity *K_{D}* value of the TCR binding to the HLA-A*02:01/SLLMWITQC is 0.39-9.3 µM, preferably the affinity *K_{D}* is 0.81-3.2 µM; such as 2.1 µM, 3.2 µM, 3.9 µM, 4.3 µM, or 4.9 µM;
the affinity *K_{D}* of the TCR binding to the HLA-A*02:16/SLLMWITQC is preferably 0.16-23 µM, such as 4.1 µM, 5 µM, 5.2 µM, 6.1 µM, 8.2 µM, 11 µM, or 15 µM, more preferably 1.3-12 µM;
the affinity *K_{D}* value of the TCR binding to the HLA-A*02:03/SLLMWITQC is preferably 1.1-76 µM, such as 6.6 µM, 8.3 µM, 13 µM, 16 µM, 18 µM, 21 µM, 22 µM, or 24 µM, more preferably 2.3-9 µM;
the affinity *K_{D}* value of the TCR binding to the HLA-A*02:09/SLLMWITQC is preferably 0.1-8.3 µM, such as 6.4 µM, more preferably 0.51-5.6 µM; further more preferably 0.57-3 µM, such as 1.4 µM, 1.7 µM or 2.4 µM;
the affinity K_{D} value of the TCR binding to the HLA-A*02:12/SLLMWITQC is preferably 0.88-85 µM, more preferably 3-35 µM; further more preferably 5.1-10 µM [The affinities described above are determined by Surface Plasmon Resonance, which is abbreviated as SPR].

Preferably, the TCR can specifically recognize and bind to the HLA-A*02:09/SLLMWITQC and/or the HLA-A*02:12/SLLMWITQC; or, the TCR can specifically recognize and bind to the HLA-A*02:03/SLLMWITQC and/or the HLA-A*02:16/SLLMWITQC.

The increase of the binding affinity of natural TCRs usually decreases the specificity of TCRs to their peptide-MHC ligands, which is confirmed in Zhao Yangbing et al., The Journal of Immunology, The American Association of Immunologists, US, vol. 179, No. 9, November 1, 2007, 5845-5854. However, the TCR in the present invention maintains the specificity to the complex, albeit with a higher binding affinity.

More preferably, the amino acid sequences of CDR1, CDR2, and CDR3 of the TCR α-chain variable region are as shown in SEQ ID NO: 71, SEQ ID NO: 72, and SEQ ID NO: 1 respectively, or as shown in derived sequences of SEQ ID NO: 71, SEQ ID NO: 72, and SEQ ID NO: 1 respectively; wherein:
the derived sequence of SEQ ID NO: 71 has 89% or more, 90% or more, 91% or more, 92% or more, 93% or more, 94% or more, 95% or more, 96% or more, 97% or more, 98% or more, or 99% or more identity to the sequence as shown in SEQ ID NO: 71.

The derived sequence of SEQ ID NO: 72 has 89% or more, 90% or more, 91% or more, 92% or more, 93% or more, 94% or more, 95% or more, 96% or more, 97% or more, 98% or more, or 99% or more identity to the sequence as shown in SEQ ID NO: 72.

The derived sequence of SEQ ID NO: 1 has 55% or more identity to the sequence as shown in SEQ ID NO: 1, preferably 66.67% or more, more preferably 77.78% or more, further more preferably 88.89% or more. The derived sequence of SEQ ID NO: 1 is preferably obtained by 1, 2, 3 or 4 point mutations in the sequence as shown in SEQ ID NO: 1, more preferably, the derived sequence of SEQ ID NO: 1 is the amino acid sequence in which at least an substitution of amino acid occurs at position 2 of the sequence as shown in SEQ ID NO: 1, the amino acid after the substitution is aromatic amino acid; according to common knowledge in the art, the aromatic amino acid comprises tyrosine (Y), phenylalanine (F), and tryptophan (W). In the present invention, in order to enhance the affinity of the TCR, the aromatic amino acid is preferably tyrosine (Y) or phenylalanine (F). In order to further enhance the affinity of the TCR, the substitution in the amino acid sequence as shown in SEQ ID NO: 1 preferably further occurs at positions 1, 4 and/or 5; more preferably, when the amino acid at position 2 after the substitution is tyrosine (Y), the substitution in the amino acid sequence as shown in SEQ ID NO: 1 further occurs at positions 1, 4 and/or 5; when the amino acid at position 2 after the substitution is phenylalanine (F), the substitution in the amino acid sequence as shown in SEQ ID NO: 1 further occurs at position 5; for position 1, the amino acid after the substitution is preferably valine (V); for position 4, the amino acid after the substitution is preferably glutamic acid (E), valine (V) or alanine (A); for position 5, the amino acid after the substitution is preferably histidine (H), asparagine (N), tryptophan (W) or alanine (A). Optimally, the derived sequence of SEQ ID NO: 1 is as shown in any one of SEQ ID NOs: 3-13 in sequence listing.

In the present invention, the amino acid sequences of CDR1, CDR2, and CDR3 of the TCR β chain variable region are shown in SEQ ID NO: 73, SEQ ID NO: 74, and SEQ ID NO: 2 respectively, or shown in derived sequences of SEQ ID NO: 73, SEQ ID NO: 74, and SEQ ID NO: 2 respectively; preferably:
the derived sequence of SEQ ID NO: 73 has 89% or more, 90% or more, 91% or more, 92% or more, 93% or more, 94% or more, 95% or more, 96% or more, 97% or more, 98% or more, or 99% or more identity to the sequence as shown in SEQ ID NO: 73.

The derived sequence of SEQ ID NO: 74 has 89% or more, 90% or more, 91% or more, 92% or more, 93% or more, 94% or more, 95% or more, 96% or more, 97% or more, 98% or more, or 99% or more identity to the sequence as shown in SEQ ID NO: 74.

The derived sequence of SEQ ID NO: 2 has 70% or more identity to the sequence as shown in SEQ ID NO: 2, preferably 80% or more, more preferably 77.78% or more, further more preferably 90% or more.

The derived sequence of SEQ ID NO: 2 is preferably obtained after 2 or 3 point mutations of the sequence as shown in SEQ ID NO: 2;

Further more preferably, the derived sequence of SEQ ID NO: 2 is the amino acid sequence in which at least a substitution of amino acid occurs at position 7 on the sequence as shown in SEQ ID NO: 2, in order to enhance the affinity of the TCR, the amino acid after the substitution is preferably asparagine (N); in order to further enhance the affinity of the TCR, the substitution in the amino acid sequence as shown in SEQ ID NO: 2 preferably further occurs at position 6, the amino acid after the substitution is preferably serine (S) or alanine (A). The substitution in the amino acid sequence as shown in SEQ ID NO: 2 preferably further occurs at position 4, the amino acid after the substitution is preferably histidine (H) or arginine (R). Preferably, when the amino acid at position 6 after the substitution is serine (S), the amino acid after the substitution for position 4 is arginine (R); when the amino acid at position 6 after the substitution is alanine (A), the amino acid after the substitution for position 4 is histidine (H). More preferably, the derived sequence of SEQ ID NO: 2 is as shown in any one of SEQ ID NOs: 14-17 in sequence listing.

The TCR as described above, preferably, the amino acid sequence of CDR3 of the TCR α chain variable region is shown in SEQ ID NO: 3, and the amino acid sequence of CDR3 of the TCR β chain variable region is shown in SEQ ID NO: 2; the amino acid sequence of CDR3 of the TCR α chain variable region is shown in SEQ ID NO: 4, and the amino acid sequence of CDR3 of the TCR β chain variable region is shown in SEQ ID NO: 2; the amino acid sequence of CDR3 of the TCR α chain variable region is shown in SEQ ID NO: 5, and the amino acid sequence of CDR3 of the TCR β chain variable region is shown in SEQ ID NO: 2; the amino acid sequence of CDR3 of the TCR α chain variable region is shown in SEQ ID NO: 6, and the amino acid sequence of CDR3 of the TCR β chain variable region is shown in SEQ ID NO: 2; the amino acid sequence of CDR3 of the TCR α chain variable region is shown in SEQ ID NO: 1, and the amino acid sequence of CDR3 of the TCR β chain variable region is shown in SEQ ID NO: 14; the amino acid sequence of CDR3 of the TCR α chain variable region is shown in SEQ ID NO: 1, and the amino acid sequence of CDR3 of the TCR β chain variable region is shown in SEQ ID NO: 15; the amino acid sequence of CDR3 of the TCR α chain variable region is shown in SEQ ID NO: 1, and the amino acid sequence of CDR3 of the TCR β chain variable region is shown in SEQ ID NO: 16; the amino acid sequence of CDR3 of the TCR α chain variable region is shown in SEQ ID NO: 1, and the amino acid sequence of CDR3 of the TCR β chain variable region is shown in SEQ ID NO: 17; the amino acid sequence of CDR3 of the TCR α chain variable region is shown in SEQ ID NO: 7, and the amino acid sequence of CDR3 of the TCR β chain variable region is shown in SEQ ID NO: 2; the amino acid sequence of CDR3 of the TCR α chain variable region is shown in SEQ ID NO: 8, and the amino acid sequence of CDR3 of the TCR β chain variable region is shown in SEQ ID NO: 2; the amino acid sequence of CDR3 of the TCR α chain variable region is shown in SEQ ID NO: 9, and the amino acid sequence of CDR3 of the TCR β chain variable region is shown in SEQ ID NO: 2; the amino acid sequence of CDR3 of the TCR α chain variable region is shown in SEQ ID NO: 12, and the amino acid sequence of CDR3 of the TCR β chain variable region is shown in SEQ ID NO: 2; or, the amino acid sequence of CDR3 of the TCR α chain variable region is shown in SEQ ID NO: 13, and the amino acid sequence of CDR3 of the TCR β chain variable region is shown in SEQ ID NO: 2.

The conventional variable regions of TCR α chain and TCR β chain in the art consist of three CDR regions and four FR regions, with the order from amino terminus to carboxyl terminus being: FR1, CDR1, FR2, CDR2, FR3, CDR3, and FR4. The TCR α chain variable region in the present invention may further comprises one or more of CDR1, CDR2, FR1, FR2, FR3, and FR4; the TCR β chain variable region in the present invention may further comprises one or more of CDR1, CDR2, FR1, FR2, FR3, and FR4;
preferably, the CDR1, the CDR2, the FR1, the FR2, and the FR3 in TCR α chain variable region are originated from germline TRAV17 or a mutant thereof, and/or the FR4 is originated from germline TRAJ-31 or a mutant thereof; the CDR1, the CDR2, the FR1, the FR2, and the FR3 in TCR β chain variable region are originatde from germline TRBV12-4 or a mutant thereof, and/or the FR4 is originated from germline TRBJ2-2 or a mutant thereof.

According to common knowledge in the art, the described mutation which mainly occurs in the framework region of TCR α chain variable region and TCR β chain variable region, is usually applied for the artificially prepared TCR on *in vitro* refolding or stability tests, especially the single chain (scTCR-wt) and soluble TCR (*i.e.:* extracellular membrane domains of TCR that may bind to other molecules such as anti-D3 antibodies, to redirect T cell), and as well the scTCR that referred as a preferred example in the present invention (TCR α chain variable region and TCR β chain variable region are connected by short peptide linkers).

More preferably:
when the CDR1, the CDR2, the FR1, the FR2 and the FR3 in TCR α chain variable region are originated from germline TRAV17 and the FR4 is originated from germline TRAJ-31, the amino acid sequence of the TCR α chain variable region is indicated as any one of SEQ ID NOs: 18-29; when the CDR1, the CDR2, the FR1, the FR2 and the FR3 in TCR β chain variable region are originated from germline TRBV12-4 and the FR4 is originated from germline TRBJ2-2, the amino acid sequence of the TCR β chain variable region is indicated as any one of SEQ ID NOs: 42-46;
Or, when the CDR1, the CDR2, the FR1, the FR2 and the FR3 in TCR α chain variable region are originated from the mutant of germline TRAV17 and the FR4 is originated from the mutant of germline TRAJ-31, the mutation sites are located in the sequence shown in SEQ ID NO: 18. According to IMGT rules, the mutation site is preferably picked at one or more of positions on 21, 45, 46, 90, 96, 126, and 128. Wherein, position 21 is preferably mutated to isoleucine (I); position 45 is preferably mutated to aspartic acid (D); position 46 is preferably mutated to proline (P); position 90 is preferably mutated to glutamic acid (E); position 94 is preferably mutated to threonine (T); position 96 is preferably mutated to proline (P); position 126 is preferably mutated to threonine (T); position 128 is preferably mutated to asparagine (N). In a preferred embodiment of the present invention, the amino acid sequence of the TCR α chain variable region is indicated as any one of SEQ ID NO: 30-41.

When the CDR1, the CDR2, the FR1, the FR2, and the FR3 in TCR β chain variable region are originated from the mutant of germline TRBV12-4 and the FR4 is originated from the mutant of germline TRBJ2-2, the mutation sites are located in the sequence shown in SEQ ID NO: 42. According to IMGT rules, the mutation site is preferably picked at one or more of positions on 5, 11-15, 45-47, 86, and 128. Wherein, position 5 is preferably mutated to (T); position 11 is preferably mutated to isoleucine (I); position 12 is preferably mutated to threonine (T); position 13 is preferably mutated to valine (V); position 14 is preferably mutated to proline (P); position 15 is preferably mutated to glutamine (Q); position 45 is preferably mutated to aspartic acid (D); position 46 is preferably mutated to proline (P); position 47 is preferably mutated to glycine (G); position 86 is preferably mutated to histidine (H); position 128 is preferably mutated to asparagine (N). In a preferred embodiment of the present invention, the amino acid sequence of the TCR β chain variable region is indicated as any one of SEQ ID NO: 47-51.

The TCR in the present invention may be in the form of an αβ heterodimer or a single chain. The single chain form comprises an αβ TCR polypeptide with the types of Vα-L-Vβ, Vβ-L-Vα, Vα-Cα-L-Vβ, or Vα-L-Vβ-Cβ (wherein Vα and Vβ are variable domains of TCR α and TCR β respectively, Cα and Cβ are constant domains of TCR α and TCR β respectively, L is the linker sequence).

The TCR is preferably an scTCR, wherein the variable region from TCR α chain and TCR β chain are connected by a linker. The sequences of the linker may be conventional in the art, which is usually applied both in antibody and TCR engineering. The common linker sequences in TCR field include (GGGSE)₄GGTG (Immunology, 2018, 155, 123; CN109400696A), GSADDAKKDAAKKDGKS *(*PNAS, 1992, 89, 4759; PNAS, 2005, 102, 19033; Protein Eng. 2011, 24, 361; WO2011044186A1), PGGG-(SGGGG)₅-P (WO2004033685A1), in which P(GGGGS)₃ is preferably *(*PNAS, 1994, 91, 12654; Cancer Gene Ther. 2004, 11, 487).

It is worth noting that the determination of the positions of CDR1-CDR3 and FR1-FR4 on the full-length sequence of TCR in the present invention is defined according to the IMGT (International Immunogenetics Information System) numbering scheme, which is well known and may be found in IMGT public data. "T cell Receptor Factsbook, (2001) LeFranc and LeFanc, Acdamic Publishing, ISBN 0-12-441352-8" discloses the sequences defined by IMGT numbering scheme as well. Specifically, FR1, CDR1, FR2, CDR2, FR3, CDR3, and FR4 of TCR α chain in the present invention are located respectively at positions 1-26, 27-38, 39-55, 56-65, 66-104, 105-117, and 118-128 on the sequence of TCR α chain variable region as shown in SEQ ID NO: 18; FR1, CDR1, FR2, CDR2, FR3, CDR3, and FR4 of TCR β chain are located respectively at positions 1-26, 27-38, 39-55, 56-65, 66-104, 105-117, and 118-128 on the sequence of TCR β chain variable region as shown in SEQ ID NO: 42,.

The conventional TCR α chain and TCR β chain in the art consist of a variable region and a constant region. The TCR α chain and/or the TCR β chain of the TCR in the present invention may further comprise a constant region, wherein the constant region of TCR α chain is originated from the germline TRAC; and/or the constant region of TCR β chain is originated from the germline TRBC2; wherein, the linkage of TCR α chain has two amino acids P and N, which is included in the natural TCRα chain. Preferably, the TCR further comprises an extra-membrane region and a transmembrane region; more preferably, the TCR further comprises an intracellular sequence.

Unless stated otherwise, the N terminal of the amino acid sequence of the TCR in the present invention is methionine residue. As is well known to those skilled in the art, the methionine will be removed during the production of recombinant protein.

The present invention also provides a nucleic acid encoding the TCR as described above.

The present invention also provides a vector comprising the nucleic acid as described above; the virus is preferably a lentiviral vector; the nucleic acid encodes a TCR α chain and a TCR β chain in a single open reading frame, or in two different open reading frames, respectively.

The present invention also provides a cell comprising the nucleic acid as described above or the vector as described above; preferably, the cell is a T cell or a stem cell, the T cell is preferably a CD8⁺ T cell.

The present invention also provides an isolated or non-naturally occurred cell, presenting the TCR as described above, the cell is preferably a T cell.

The present invention also provides a pharmaceutical composition comprising the TCR as described above or the cell as described above; preferably, the pharmaceutical composition further comprises a pharmaceutically acceptable carrier.

The present invention also provides a kit comprising the TCR as described above, the nucleic acid as described above, the cell as described above, or the pharmaceutical composition as described above; the kit may further comprise other pharmaceutically active ingredient which acts synergistically with the TCR, the nucleic acid, the cell, or the pharmaceutical composition of the present invention.

The present invention also provides a use of the TCR as described above, the cell as described above, or the pharmaceutical composition as described above in the manufacture of a medicament for preventing and/or treating a NY-ESO-1 expression-associated tumor; preferably, the NY-ESO-1 expression-associated tumor comprises synovial sarcoma, liposarcoma, myeloid malignant leukemia, malignant melanoma, ovarian cancer, neuroblastoma, prostate cancer, bladder cancer, breast cancer, hepatocellular carcinoma, non-small cell lung cancer, oral squamous carcinoma, and esophageal cancer.

The present invention also provides a method of preventing and/or treating a NY-ESO-1 expression-associated tumor comprising administering the TCR as described above, the nucleic acid as described above, the cell as described above, or the pharmaceutical composition as described above to a subject in need thereof; preferably, the tumor comprises synovial sarcoma, liposarcoma, myeloid malignant leukemia, malignant melanoma, ovarian cancer, neuroblastoma, prostate cancer, bladder cancer, breast cancer, hepatocellular carcinoma, non-small cell lung cancer, oral squamous carcinoma, and esophageal cancer.

Note: in the present invention, the numbers "1" or "2" behind the terms, such as "1" and "2" in *K_{D1}* and *K_{D2},* indicate no practical meaning and are only used to distinguish the same terms.

Based on the common knowledge in the art, each of the above preferred conditions can be combined arbitrarily to obtain any preferred examples of the present invention.

The reagents and raw materials used in the present invention are commercially available.

The positive and progressive effects of the present invention are that:

The TCR of the present invention exhibit extremely high affinity and excellent safety, with the affinity *K_{D1}* of 0.1-10 µM against HLA-A*02:01/SLLMWITQC; in addition, the TCR of the present invention is able to bind to other complexes such as HLA-A*02:03/SLLMWITQC, HLA-A*02:09/SLLMWITQC, HLA-A*02:12/SLLMWITQC, and HLA-A*02:16/SLLMWITQC, with the affinity K_{D2} up to 0.1-85 µM, or even 0.57-27 µM, possessing a wider applicability. Furthermore, these mutants exhibit specific killing activity against various tumor cell lines after transduction into CD8⁺ T cells via lentivirus, indicating a good prospect of application.

### BRIEF DESCRIPTION OF THE DRAWINGS

FIG. 1 shows the double-positive clone cells detected by sorting.
FIG. 2A and FIG. 2B show the anion exchange chromatography and SDS-PAGE of NY^{c9} scTCR-wt after the refolding, wherein the refolding was failed.
FIG. 3A and FIG. 3B show the anion exchange chromatography and SDS-PAGE of NY^{c9} scTCR_X0 after the refolding.
FIG 4A and FIG. 4B show the gel filtration chromatography and SDS-PAGE of NY^{c9} scTCR_X0 after the refolding, wherein the purification was succeeded.
FIG. 5 shows the affinity assay of NY^{c9} scTCR_X0 and HLA-A*02:01/SLLMWITQC.
FIG. 6 shows the purification graph of inclusion bodies.
FIG. 7A and FIG. 7B show the anion exchange chromatography and non-reducing SDS-PAGE of NY^{c9} A5B0.
FIG. 8A and FIG. 8B show the gel filtration chromatography and SDS-PAGE of NY^{c9} A5B0.
FIG. 9A and FIG. 9B show the anion exchange chromatography and SDS-PAGE of HLA-A*02:01/β2M/SLLMWITQC after the refolding.
FIG. 10A and FIG. 10B show the gel filtration chromatography and SDS-PAGE of HLA-A*02:01/β2M/SLLMWITQC; wherein, the band with large molecular weight is HLA-A*02:01, the band with small molecular weight is β2M, and the band of SLLMWTQC cannot be seen on SDS-PAGE due to its small molecular weight.
FIG. 11 shows Gel Shift graph of pMHC after biotinylation.
FIG. 12 shows the affinity assay graph of NY^{c9} A5B0 to HLA-A*02:01/SLLMWITQC.
FIG. 13 shows the effects of lentivirus infection of NY^{c9} A5B0.
FIG. 14 shows the INF-γ release graphs of NY^{c9} A2B0, A3B0 and A5B0.
FIG. 15 shows the specific LDH killing assay of tumor cell lines.
FIG. 16 shows the tumor growth in mice on Day 22 after the inoculation of tumor cells and T cells; the arrow points to the site where the tumor is located.
FIG. 17 shows the tumor growth curves.
FIG. 18 shows the INF-γ release data of NY^{c9} A5B0 on 10 cases of PBMC with HLA-A*02:01 typing from healthy individuals.
FIG. 19 shows the INF-γ release data of NY^{c9} A5B0 on 30 cases of PBMC with non-HLA-A*02:01 typing from healthy individuals.
FIG. 20 shows the INF-γ release data of NY^{c9} A5B0 on 5 cases of embryonic progenitor cells.
FIG. 21 shows the INF-γ release data of NY^{c9} A5B0 against different HLA-A*02:0x typing.
FIG. 22 shows TCR-T cells transduced with NY^{c9} A5B0, which cannot significantly recognize human astrocytes (HA) transfected with different HLA-A*02:0x typing.
FIG.23 shows TCR-T cells transduced with NY^{c9} A5B0, which cannot significantly recognize human lung fibroblasts (HLF) transfected with different HLA-A*02:0x typing.
FIG. 24 shows TCR-T cells transduced with NY^{c9} A5B0, which cannot significantly recognize human embryonic liver cells (CCC-HEL) transfected with different HLA-A*02:0x typing.
FIG. 25 shows TCR-T cells transduced with NY^{c9} A5B0, which cannot significantly recognize human embryonic pulmonary fibroblasts (CCC-HPF) transfected with different HLA-A*02:0x typing.
FIG. 26A-FIG. 26P show the sequences of lentiviral vectors expressing A0B0, A1B0, A2B0, A3B0, A4B0, A5B0, A6B0, A7B0, A8B0, A9B0, A10B0, A11B0, AOB1, A0B2, A0B3, and A0B4, respectively.

### DETAILED DESCRIPTION OF THE EMBODIMENT

The present dislcosure is further illustrated below by way of embodiments, but the present invention is not thereby limited to the scope of the described embodiments. Experimental methods for which specific conditions are not indicated in the following examples are selected according to conventional methods and conditions, or according to the product instructions.

TCRs of the present invention were obtained by the following experimental processes:
1. Specific CD8⁺ T cell was cloned to obtain heterodimeric TCR sequence;
2. Stability modification on the scTCR domain of this TCR was performed;
3. The modified scTCR sequence was subject to perform phage display to obtain CDR3α, CDR3β sequence mutations with high affinity;
4. These mutations were introduced into the heterodimeric TCR to prepare the corresponding TCR *in vitro,* then their affinity to pMHC of the specific target HLA*A02:01/SLLMWITQC was determined. The *K_{D}* value was required to be in the range of 0.1 to 10 µM;
5. A series of mutants specifically targeting HLA-A*02:01/SLLMWITQC were prepared, and their affinity was further tested to obtain heterodimeric TCR mutants with good specificity and high affinity from the biochemical aspect;
6. The heterodimers with good specificity and high affinity were further prepared into lentiviruses to infect CD8⁺ T cells, which are capable of recognizing the tumor cells specifically expressing HLA-A*02:01/SLLMWITQC;
7. The transduced CD8⁺ T cells were used to perform *in vitro* specific killing assays on tumor cell lines to obtain heterodimeric TCR mutated sequences that may effectively and specifically kill tumor cells.

After the TCRs described above were obtained, *in vivo* tumor killing assays were performed in animals.

Details are described in the following examples.

The following examples take NY^{c9} A5B0 (NY refers to the protein name NY-ESO-1, and c9 refers to the monoclonal CD8⁺ T cells grown from this well) as an example to describe the preparation process of the TCR of the present invention in detail.

In addition: In the following examples, the cell lines used were purchased from ATCC unless otherwise stated.

### Example 1 Cloning the specific CD8⁺ T cell to obtain heterodimeric TCR sequence (acquisition and identification of wild-type TCR)

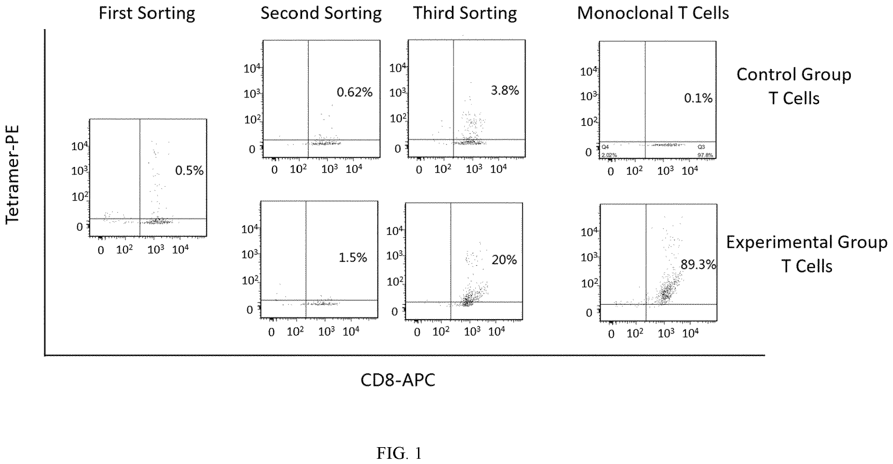

The methods, reagents, and materials used for T-cell cloning are primarily referenced in Curr. Protoc. Immunol. 2002, 7, 1; PLoS One, 2011, 6, e27930; Onco Immunology 2016, 5, e1 175795 and references therein. B cells (EBV-B) were generated by EBV (EB virus) infection and NY-ESO-1 short peptide (SLLMWITQC) loading *(*J Vis Exp. 2011, 8, 3321) (EBV virus: ATCC Item Code VR-1492), which were used to stimulate the CD8⁺ T cells from healthy donors with HLA-A*02:01 genotype. Here, the culture methods of monoclonal T cells mainly reference to the work of the relevant literatures (J Immunol Methods. 2006, 310, 40; PLoS One. 2014, 9, e110741). Double-positive T cells were sorted via the PE-labeled short peptide-HLA tetramer (MBL, Cat. No. TS-M047-1) and the APC-labeled anti-CD8 antibody (Biolegend, Cat. No. 301014). The sorting was performed when T cells were proliferated to 5,000-10,000 cells after the stimulation with SLLMWITQC short peptide. After stimulation, culture and sorting for 2-3 rounds, the cells were limitedly diluted to about 0.5 cell/well and cultured to obtain monoclones (with reference to PhD thesis of Department of Pharmacy in Cardiff University, Lissina A., 2011. Optimisation of T cell receptor antigen recognition for targeting disease). The proliferated monoclonal T cells were used for subsequent tetrameric staining sorting. As shown in FIG. 1, the total mRNA of sorted double-positive monoclonal T cells were extracted with the kit Quick-RNA^{™} MiniPrep (ZYMO research, Cat. No. R1050). The achieved mRNA was further reversely transcribed into cDNA using SMART RACE cDNA amplification kit of clontech, which was subsequently cloned to pUC19 (Invitrogen, Product No. SD0061) for sequencing.

The wild-type TCR was finally obtained, and after the sequence identification and analysis, the full-length compositions of its α chain and β chain were obtained as follows:

Full length of α chain:

METLLGVSLVILWLQLARVNSQQGEEDPQALSIQEGENATMNCSYKTSINNL
QWYRQNSGRGLVHLILIRSNEREKHSGRLRVTLDTSKKSSSLLITASRAADTASYFCA
SDQDARLMFGDGTQLVVKPNIQNPDPAVYQLRDSKSSDKSVCLFTDFDSQTNVSQ
SKDSDVYITDKTVLDMRSMDFKSNSAVAWSNKSDFACANAFNNSIIPEDTFFPSP
ESSCDVKLVEKSFETDTNLNFQNLSVIGFRILLLKVAGFNLLMTLRLWSS (SEQ ID NO: 52).

Full length of β chain:

MDSWTLCCVSLCILVAKHTDAGVIQSPRHEVTEMGQEVTLRCKPISGHDYL
FWYRQTMMRGLELLIYFNNNVPIDDSGMPEDRFSAKMPNASFSTLKIQPSEPRDSAV
YFCASSLGPGELFFGEGSRLTVLEDLKNVFPPEVAVFEPSEAEISHTQKATLVCLAT
GFYPDHVELSWWVNGKEVHSGVSTDPQPLKEQPALNDSRYCLSSRLRVSATFW

QNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 53).

In the full length sequences of α chain or β chain as described above: the underlined portion is the signal peptide sequence, the unlabeled portion is the sequence of Vα (variable region of α chain; the sequence is shown in SEQ ID NO: 18) or the sequence of Vβ (variable region of (3 chain; the sequence is shown in SEQ ID NO: 42), the bolded portion is the sequence of Cα (constant region of α chain) or the sequence of Cβ (constant region of β chain), the italicized portion is the extracellular linker, the italicized and underlined portion is the sequence of transmembrane region and intracellular region. According to the IMGT rule, FR1, CDR1 (SEQ ID NO: 71), FR2, CDR2 (SEQ ID NO: 72), FR3, CDR3 (SEQ ID NO: 1), and FR4 of TCR α chain are located respectively at positions 2-27, 28-32, 33-49, 50-56, 57-90, 91-99 and 100-109 within the sequence of TCR α chain variable region which is shown in SEQ ID NO: 18. The FR1, CDR1 (SEQ ID NO: 73), FR2, CDR2 (SEQ ID NO: 74), FR3, CDR3 (SEQ ID NO: 2), and FR4 of TCR β chain are located respectively at positions 1-26, 27-31, 32-48, 49-54, 55-92, 93-102, and 103-112 within the sequence of TCR β chain variable region which is shown in SEQ ID NO: 42.

The amino acid sequence of A0 as shown in SEQ ID NO: 54 and the amino acid sequence of B0 as shown in SEQ ID NO: 66 were obtained by removing the full-length transmembrane region and intracellular sequence (italicized and underlined portion) from α chain and β chain, respectively (wherein "A" and "B" represent the α chain and β chain, respectively, and "0" represents no mutation).

### Example 2 Stability modification to the scTCR domain of the wild-type TCR prepared in Example 1

The Vα and Vβ (the full length variable region of α chain and β chain are shown in SEQ ID NO: 18 and SEQ ID NO: 42, respectively) obtained in Example 1 were connected together using Linker amino acid sequence to generate the wild-type scTCR (hereinafter referred as scTCR-wt), the structure of which is very unstable and requires stability modification for subsequent affinity optimization *(*PNAS, 1999, 96, 5651; Nat Biotechnol., 2000, 18, 754; Front. Oncol., 2015, 4, 1; WO2016124142) and inclusion body refolding (cloning, expression, and purification processes of scTCR-wt were performed according to conventional methods in the art).

For the β-turn structure of scTCR-wt, mutations that contribute to the stability of β-turn structure were introduced according to the preference of different amino acid side chains for different positions of the β-turn. Due to the removal of Cα and Cβ regions, hydrophobic amino acids at some positions of Vα and Vβ domains are exposed to the surface. The stable scTCR_X0 sequence has been achieved via the reduction of amino acid mutation with surface hydrophobic properties. Genes of scTCR-wt and scTCR-X0 were directly synthesized and then cloned into the pET28a vector by Nco I/Not I endonuclease.

The stability optimized scTCR_X0 has mutations in the FR1, FR2, FR3, and FR4 regions of TCR α and TCR β chains. As shown in SEQ ID NO: 18, the mutations are preferably at one or more positions at 21, 45, 46, 90, 94, 96, 126, and 128 thereof according to the IMGT numbering; as shown in SEQ ID NO: 42, the mutations are preferably at one or more positions at 5, 11-15, 45-47, 86 and 128 thereof according to the IMGT numbering. The variable regions were achieved after a series of mutations on A0 framework (sequence is shown in SEQ ID NO: 30) and B0 framework (sequence is shown in SEQ ID NO: 47). The variable regions generated from the mutations of A0 and B0 framework were connected by the peptide Linker sequence P(GGGGS)₃; hereinafter referred as Linker sequence or Linker) to obtain scTCR_X0 [the amino acid sequence is shown as SEQ ID NO: 30+Linker sequence+SEQ ID NO: 47 (N-terminal is on the left, C-terminal is on the right; hereinafter N on left and C on right unless stated otherwise)].

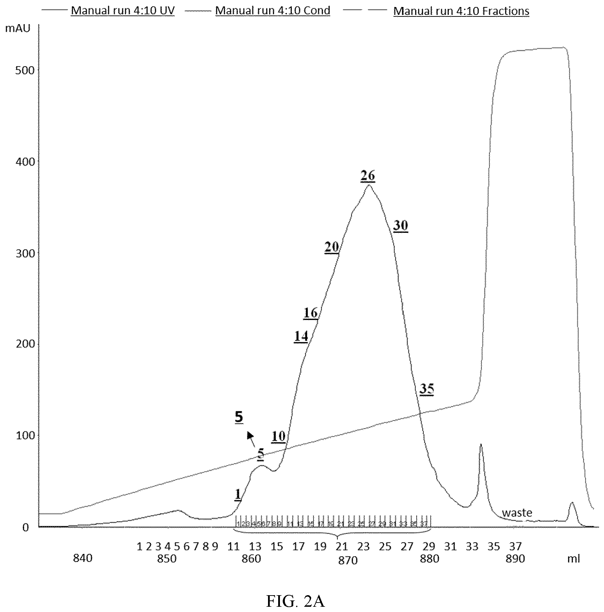

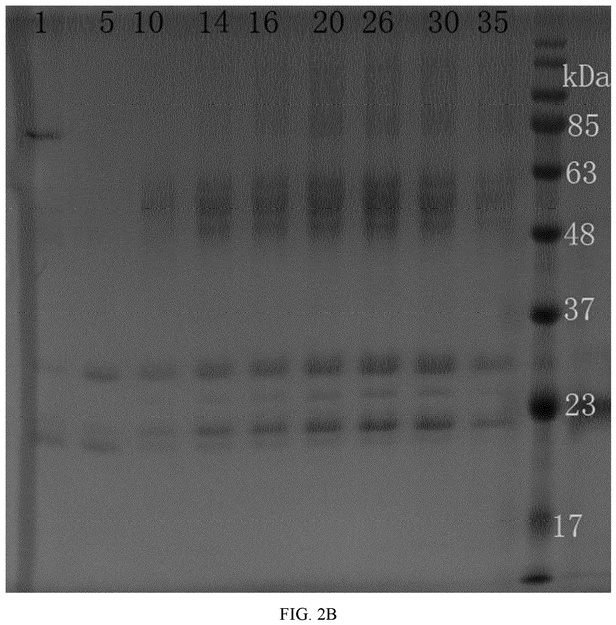

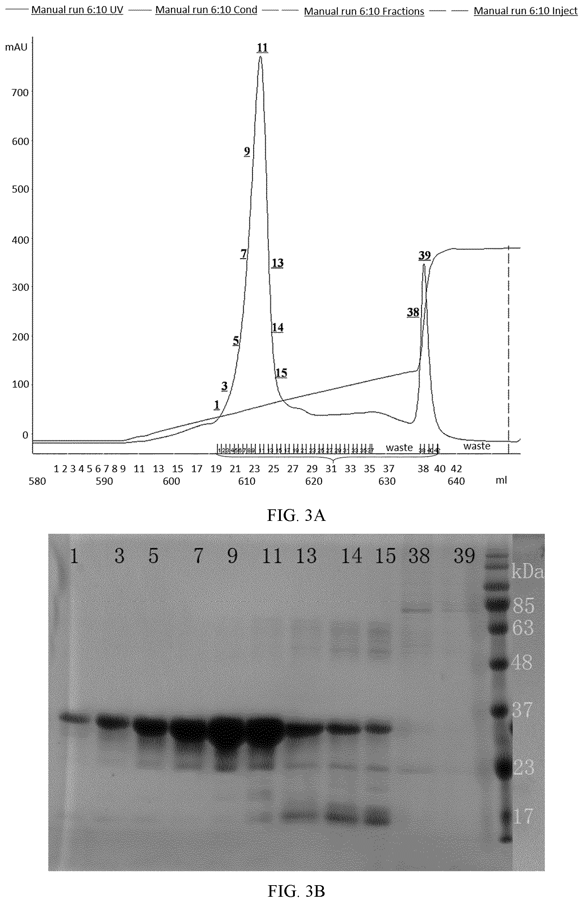

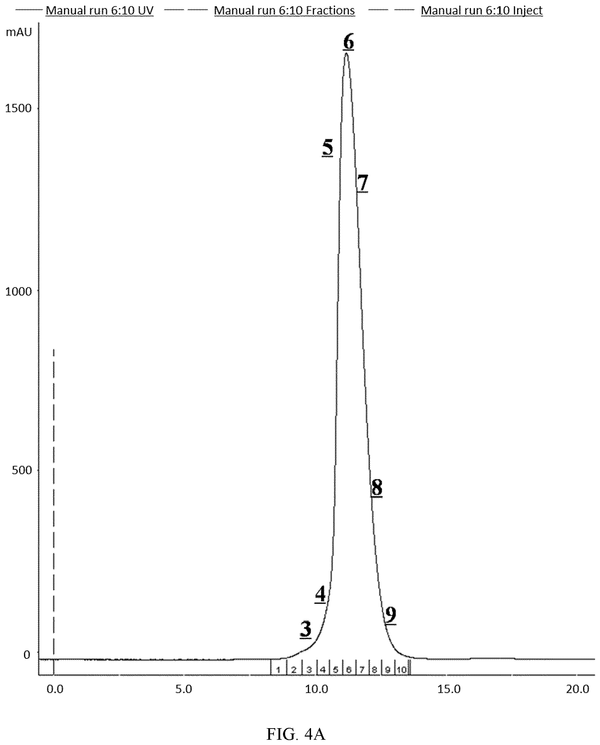

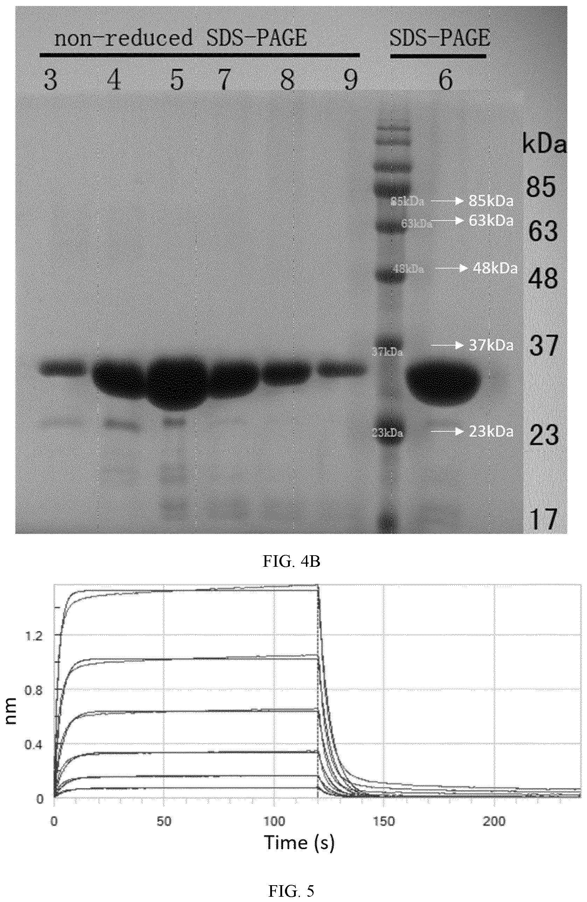

The inclusion body expression, protein refolding and purification of scTCR-wt and scTCR-X0 were performed and their affinity tests were further evaluated as described in Example 4, Example 5 and Example 7, and the results are shown in FIG. 2A, FIG. 2B, FIG. 3A, FIG. 3B, FIG. 4A, FIG. 4B, and FIG. 5.

### Example 3 Phage display

The engineered gene of scTCR_X0 was cloned into the phage display vector via the cleavage sites of Nco I/Not I, which was applied as a template for constructing phage library. Discontinuous mutant primers were designed to construct the library of CDR3α/CDR3β variable region, which were electrotransferred into TG1 competent cell for the subsequent 2-3 rounds of phage screening. Besides the classic molecular biology handbook, the overall process of phage screening mainly references to two articles Nature Protocols, 2007, 2, 3001 and Nat. Biotech. 2005, 23, 349, and a PhD thesis from Department of Pharmacy in Cardiff University (Liddy S. 2013. Molecular engineering of high affinity T-cell receptors for bispecific therapeutics).

Library screening: library bacteria were inoculated in 30-50 mL 2×YT medium containing 100 µg/mL ampicillin and 2% glucose with OD₆₀₀ value between 0.05-0.08. The bacteria were cultured at 37°C, 200-220 rpm until OD₆₀₀=0.4-0.5.

The helper phage was added at the ratio (molar ratio) of 15:1-25:1 (phage:bacteria), mixed well and kept at 37°C for 30 min in stationary. The mixture was centrifuged at room temperature with low speed for 10 min, then the supernatant was discarded. The bacteria were resuspended in 30-50 mL 2×YT medium containing 100 µg/mL ampicillin+50 µg/mL kanamycin, and cultured at 26°C, 200-220 rpm for overnight.

The bacterial culture was centrifuged at 4°C with high speed for 10 min. The supernatant was collected and mixed with PEG/NaCl solution at a ratio of 4:1. The mixture was put on ice for 1 hour, and then centrifuged at 4°C with low speed for 10 min. The precipitates were collected and resuspended with 10 ml PBS, then centrifuged at 4°C with high speed for 10 min. The supernatant was collected and added 2.5 ml PEG/NaCl, and further centritrifuged at 4°C with low speed for 30 min after being kept on ice for 20-30 min. The phage solution was obtained after resuspending the precipitates with 1 mL PBS.

The milk solution (3% w/v) was added to the proper volume of phage solution described above for 1 hour blocking. Then, an appropriate amount of biotinylation-labeled pMHC was added and reacted for 1 hour at room temperature to form the phage-pMHC complex. After the addition of 50 µl of streptavidin magnetic beads, the magnetic bead-phage-pMHC complexes were further achieved.

The magnetic beads were thoroughly washed for 3-5 rounds by magnet adsorption, then 0.1 mg trypsin was added (final concentration 1 mg/mL) and incubated for 30 min at room temperature. Via magnet adsorption, the supernatant was collected and used to infect the preactivated TG1 bacteria strain. The bacteria strain was spread on plates and incubated upside down at 30°C for overnight. The above process may be repeated for 2-3 rounds of screening according to experimental needs.

Phage ELISA assay: Referring to the workflow of Nature Protocols, 2007, 2, 3001, single clones were selected to culture at 37°C, 200-220 rpm in 150 mL 2×YT medium (100 µg/mL ampicillin and 2% glucose) for overnight. 2-5 µL bacteria solution of each clone was transferred into a new 96-well plate containing 150-200 µL of the same medium and cultured at 37°C, 200-220 rpm for 3 hours. Then 50 µL helper phage with sufficient titer was added into each well and incubated at 37°C, 200-220 rpm for 1 hour. After centrifugation with low speed for 10 min at room temperature, the precipitate was collected and resuspended in 200 µL medium (2×YT medium added with 100 µg/mL ampicillin+50 µg/mL kanamycin), which was then cultured at 26°C, 200-220 rpm for overnight.

A 96-well ELISA plate was coated by 1 µg streptavidin per well and kept at 4°C in stationary for overnight. After the ELISA plate was washed 3 times by PBS, 0.5 µg (volume concentration) biotin-labeled pMHC was added to react for 30 min at room temperature. Then 400 µL PBS containing 6% milk was added for blocking at room temperature for 1 hour.

Identical volume of milk solution was added to 100 µL fresh phage supernatant for 1 hour blocking at room temperature, 100 µL of which was then added into the PBS-washed ELISA plate to react for 1 hour at room temperature.

The ELISA plate was washed for 3-5 rounds. The diluted M13-HRP antibody (Yiqiao Shenzhou, Cat No. 11973-MM05T-H, ratio of dilution 1:10000) was added to react for 30 min at room temperature. The ELISA plate was washed again for 5 rounds, and 50 µL color-substrate solution was added into each well to develop color for 90 seconds, then the reactions were stopped immediately, followed by the determination of absorption value (OD₄₅₀) via microplate reader. With OD₄₅₀ values above 0.45, the monoclonal phages were selected to perform DNA sequencing for screening, generating the corresponding mutants of scTCR_X1-15 [the amino acid sequences thereof were shown as SEQ ID NO: 30+Linker+SEQ ID NO: 42, SEQ ID NO: 30+Linker+SEQ ID NO: 43, SEQ ID NO: 30+ Linker+SEQ ID NO: 44, SEQ ID NO: 30+Linker+SEQ ID NO: 45, SEQ ID NO: 30+Linker+SEQ ID NO: 46, SEQ ID NO: 31+Linker+SEQ ID NO: 42, SEQ ID NO: 32+Linker+SEQ ID NO : 42, SEQ ID NO: 33+Linker+SEQ ID NO: 42, SEQ ID NO: 34+Linker+SEQ ID NO: 42, SEQ ID NO: 35+Linker+SEQ ID NO: 42, SEQ ID NO: 36+Linker+SEQ ID NO: 42, SEQ ID NO: 37 +Linker+SEQ ID NO: 42, SEQ ID NO: 38+Linker+SEQ ID NO: 42, SEQ ID NO: 39+Linker+SEQ ID NO: 42, and SEQ ID NO: 40+Linker+SEQ ID NO: 42]. After sequence analysis, these mutants mentioned above are found to occur in CDR3α and CDR3β, with their specific sites shown in Table 1 of the following example. The Linker described in this example is the same as the Linker sequence in Example 2, which is P(GGGGS)₃.

### Example 4 Gene cloning and inclusion body expression

As shown in Table 1, the mutated sites obtained in Example 3 were correspondingly introduced into A0 and B0 of TCR, generating the variable regions of A1-A11 with amino acid sequences as shown in SEQ ID NO: 19-29, and the variable regions of B1-B4 with amino acid sequences as shown in SEQ ID NO: 43-46; and correspondingly obtaining TCR α chains A1-A11 with amino acid sequences as shown in SEQ ID NO: 55-65, and TCR β chains B1-B4 with amino acid sequences as shown in SEQ ID NO: 67-70, respectively ("A" represents α chain, "B" represents β chain, "A" or "B" with different numbers indicate "α chain" or "β chain" containing different mutations; additionally, the heterodimer consisting of TCR α chain and TCR β chain is abbreviated as AmBn in the following examples; wherein m is an integer from 0 to 11 and n is an integer from 0 to 4).

The mutated sites on CDR3α and CDR3β are listed in Table 1 (wherein the mutated residues are highlighted by bold):

**Table 1 Mutated sites display**

| Numbering | CDR3 | SEQ ID NO. |
|---|---|---|
| A1 | A**Y**D**A**DARLM | 3 |
| A2 | A**F**D**AH**ARLM | 4 |
| A3 | A**Y**D**EH**ARLM | 5 |
| A4 | A**Y**D**VH**ARLM | 6 |
| A5 | A**Y**DQDARLM | 7 |
| A6 | A**Y**D**EN**ARLM | 8 |
| A7 | A**Y**D**VA**ARLM | 9 |
| A8 | **GY**DQ**DA**RLM | 10 |
| A9 | **SY**DQ**E**ARLM | 11 |
| A10 | **VY**DQ**N**ARLM | 12 |
| A11 | A**Y**DQ**W**ARLM | 13 |
| B1 | ASSLG**AN**ELF | 14 |
| B2 | ASS**H**G**AN**ELF | 15 |
| B3 | ASSLG**SN**ELF | 16 |
| B4 | ASS**R**G**SN**ELF | 17 |

The expression process is shown in detail as follows:

The genes of TCR α-chain (AO-A11), β-chain (B0-B4), scTCR, HLA-A*02:01 (or HLA-A*02:03, HLA-A*02:09, HLA-A*02:12, HLA-A*02:16) and β2M (HLA-A*02:01, β2M and SLLMWITQC are refolded simultaneously to form pMHC complex; wherein UniProt ID of HLA-A*02:01 is P01892, HLA-A*02:03, HLA-A*02:09, HLA-A*02:12, HLA-A*02:16 are polymorphic mutants of HLA-A*02:01, UniProt ID of β2M is P61769) were cloned into pET28a vector (purchased from Novagen) via the Nco I/Not I cleavage site, and transformed into *E. coli* BL21 (DE3) (purchased from NEB), respectively. Single clones were cultured in LB medium at 37°C with shaking until OD₆₀₀ = 0.6-0.8, then IPTG was added to reach a final concentration of 0.8 mM. The solution was continued to culture at 37°C for 3 hours and then centrifuged at 6000 rpm for 10 min to collect bacterial pellets, which were then stored at -20°C.

### Example 5 Purification, refolding and purification of inclusion body

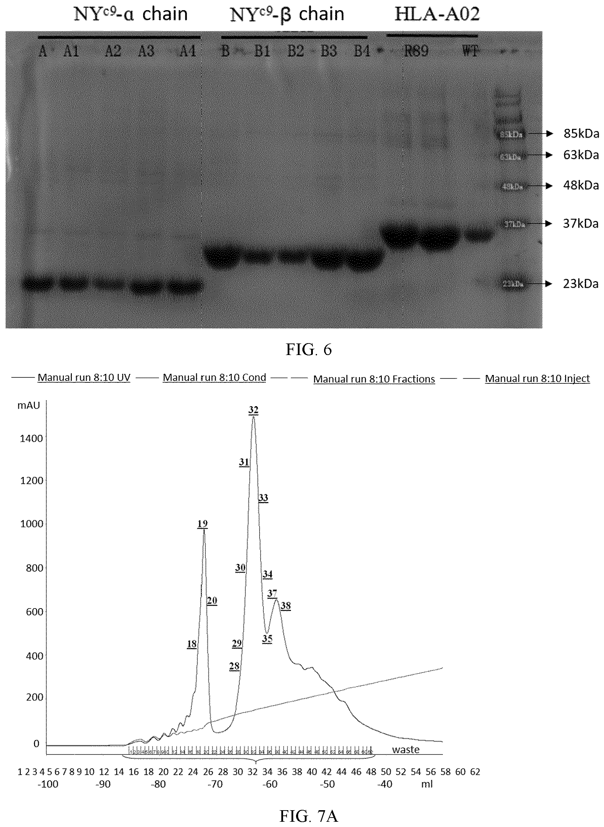

The bacterial pellets were resuspended with lysis solution (PBS containing 0.5% TritonX 100), extracted by ultrasonication and then centrifuged at high speed of 12000 rpm for 20 min. The supernatant was discarded, and the precipitates were resuspended with lysis solution until no particles are visible, then the pellets were centrifuged at high speed for 10 min. The above operations were repeated for 2-3 rounds, then the precipitates were dissolved with 6M guanidine hydrochloride solution and centrifuged at high speed for 10 min to collect the supernatant, which referred as the purified inclusion bodies. 1 µL of the supernatant was subject to perform SDS-PAGE. FIG. 6 shows that the purity of inclusion bodies satisfies the requirements. The inclusion bodies were quantified, separated, and froze for storage at -80°C.

20 mg TCR α chain and 15 mg β chain (prepared in Example 4) were diluted in a 5 mL 6M guanidine hydrochloride solution, respectively. The TCR α chain and TCR β chain were added slowly and sequentially into the pre-cooled refolding buffer (Science 1996, 274, 209; J. Mol. Biol. 1999, 285, 1831; Protein Eng. 2003, 16, 707) and mixed with continuous stirring for 30 min at 4°C. The mixture was then put into dialysis bag and dialyzed in 10 times the volume of pre-cooled deionized water with stirring for 8-12 hours. The dialysis was repeated for 2-3 rounds in the pre-cooled dialysate (pH 8.1, 20 mM Tris-HCl) at 4°C for 8 hours.

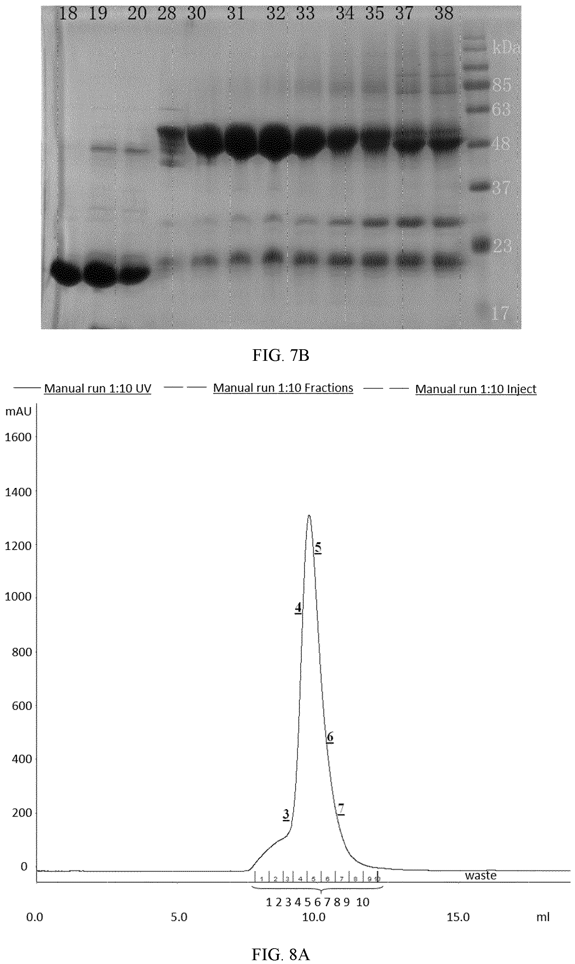

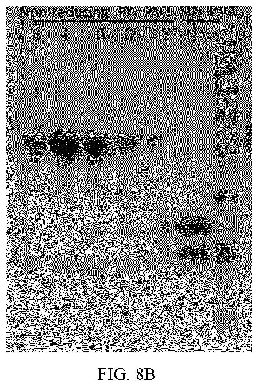

The solution was poured out from dialysis bag, centrifuged at high speed for 10 min to remove precipitation and air bubbles, and subject to anion exchange chromatography with HiTrap Q HP (5 ml). The solution was linearly eluted with 0-2 M NaCl, 20 mM Tris pH 8.1. The elution peaks containing the target protein fractions were collected, combined and concentrated. In non-reducing SDS-PAGE electrophoresis, the band near 48 kD is indicated as NY^{c9}-A5B0, but further purification was required due to the unqualified purity. The concentrated protein sample was subject to size-exclusion chromatography with superdex 75 10/300. The non-reducing SDS-PAGE showed a band near 48 kD with high concentration, while the reducing SDS-PAGE showed α-chain and β-chain two bands with a purity of about 90%, respectively,. The details are as shown in FIG. 7A, FIG. 7B, and FIG. 8A, FIG. 8B (given that the molecular weights of A0-A11 are equal and the molecular weights of B1-B4 are equal, only the purification results of A5 and B0 are shown herein as examples).

### Example 6 Preparation of biotinylated antigenic peptide-MHC (pMHC)

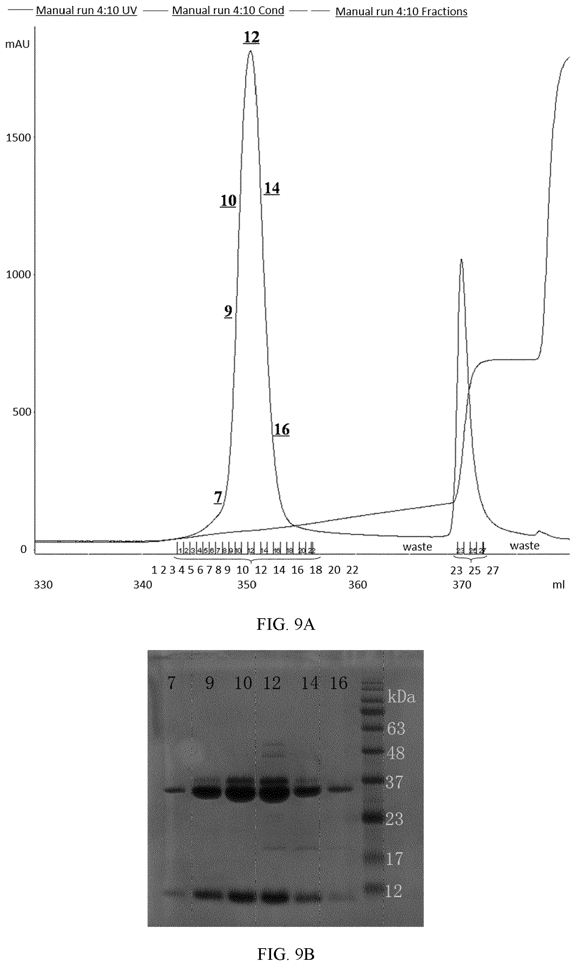

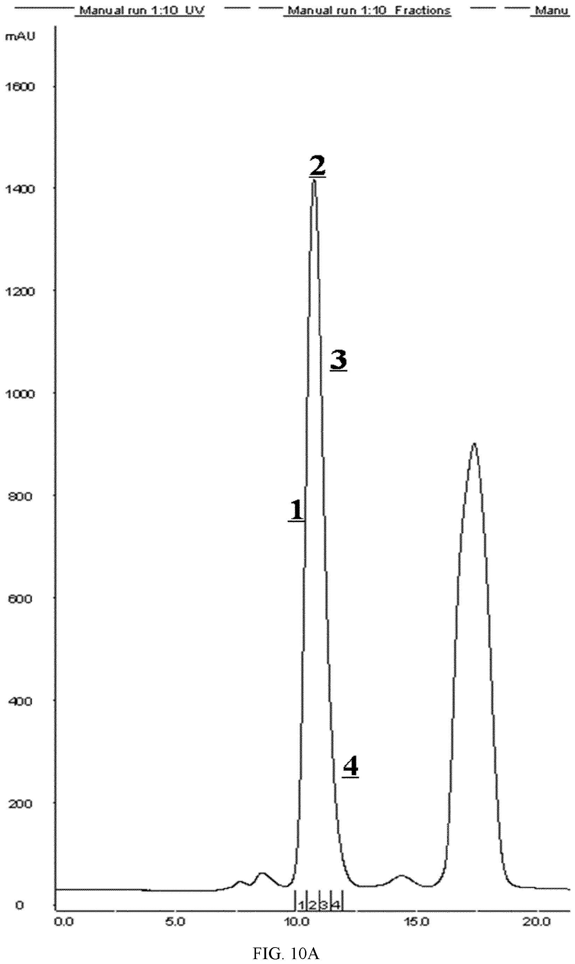

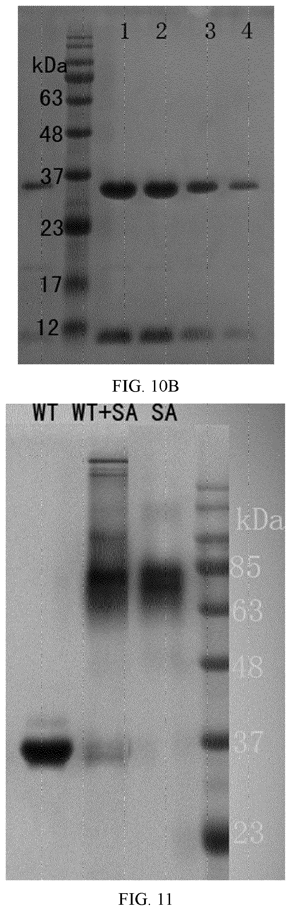

The refolding and purification of pMHC were performed according to the methods from NIH Tetramer Core Facility (http://tetramer.yerkes.emory.edu/support/protocols). Here HLA-A*02:01/SLLMWITQC is taken as a representative example. According to the online protocols, the polypeptide solution as well as the inclusion body solutions of β2M and HLA-A*02:01 were sequentially added into the refolding buffer (0.1 M Tris-HCl, 0.4 M L-arginine, 2 mM EDTA; 0.5 mM oxidative glutathione and 5 mM reduced glutathione, 0.2 mM PMSF), and stirred overnight at 4°C. An identical amount of inclusion body solution of HLA-A*02:01 was added to the mixture in the next morning and evening, respectively, and stirred at 4°C for 1-3 days. The mixture was then dialyzed in 10 times the volume of dialysate (pH 8.1, 20 mM Tris-HCl) for 3 rounds. The dialyzed protein samples were subject to anion exchange chromatography with HiTrap Q HP (5 ml) and linearly eluted with 0-2M NaCl, 20 mM Tris pH 8.1 solution. The elution peaks were collected and analyzed by SDS-PAGE electrophoresis. Two bands indicating HLA-A*02:01 and β2M respectively with higher purity can be observed in the gel graph, while the band of SLLMWITQC cannot be seen due to its small molecular weight. The elution peaks containing pMHC fraction were combined, concentrated and further purified by gel filtration chromatography (Superdex 75 10/300), then detected by SDS-PAGE. The electrophoretogram indicated that the pMHC complex with better purity was achieved. Biotinylation *(*Protein Expr. Purif. 2012, 82, 162; J. Bacteriol. 2012, 194, 1113.) was performed via recombinant enzyme BirA (BPS Bioscience product, Product No.: 70031), with the reaction system prepared according to the method of *NIH Tetramer Core Facility* and the purity determined via Gel Shift. Electrophoretogram of Gel Shift indicated that the purity satisfied the requirement. The results are detailed in FIG. 9A, FIG. 9B, FIG. 10A, FIG. 10B and FIG. 11.

### Example 7 Affinity assay

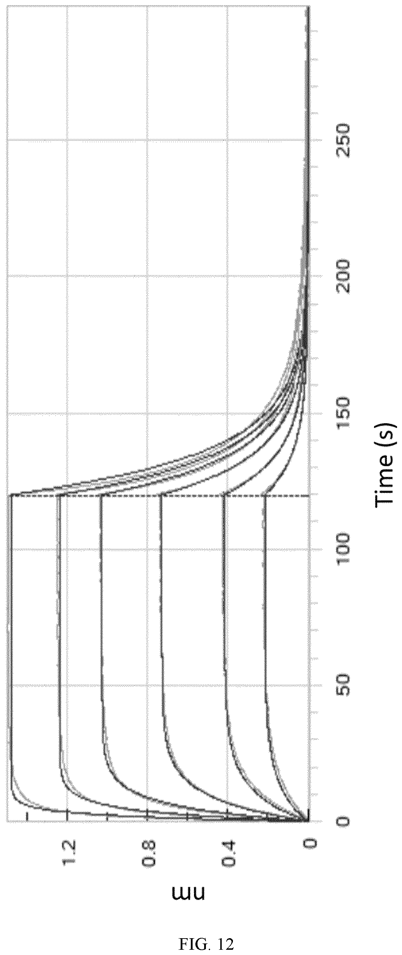

Octet is an instrument for affinity assay using SPR technology. According to the optical interference technology of biofilm layers based on fiber optic biosensors, the binding dissociation constants were calculated by detection on kinetic parameters of interacting molecules, and analysis of kinetic and affinity. In this experiment, we used SA sensors to immobilize biotinylated pMHC and detected the binding dissociation constants thereof with different TCRs to calculate the *K_{D}* values. NY^{c9} A5B0 was used as a representative example to test the affinity of HLA-A*02:01/SLLMWITQC, which is detailed in FIG. 12.

Table 2 shows a compiled summary of the affinity of NY^{c9} TCR mutants with HLA-A*02:0x/SLLMWITQC. It can be seen from the results that, for most NY^{c9} mutants, the *K_{D}* for binding to HLA-A*02:0x/SLLMWITQC is between 0.1-10 µM, preferably between 0.39-9.3 µM, and more preferably between 0.81-3.2 µM, wherein the x is 1, 3, 9, 12 and 16.

It should be noted that, as known to those skilled in the art, SPR technology is one of the most common and reliable methods for affinity assay at present, but due to the involvements of protein quantification, obsolescence degree of the chip, instrument status, etc., different batches of experiments may result in some errors, the value of which may even be 3-5 times; whereas, the same batches of experiments using the same protein quantification, chip and instrument were performed in the present invention, therefore each data may be used to compare the affinity, but its specific value does not constitute a limitation on the scope of protection for the present invention.

**Table 2 Mutated residues are highlighted by underline**

| | **CDR3α** | **CDR3β** | **A*02:01** | **A*02:03** | **A*02:09** | **A*02:12** | **A*02:16** |
|---|---|---|---|---|---|---|---|
| | | | **K_{D}(M)** | | | | |
| A1B0 | A**Y**D**A**DARLM | | 5.7E-06 | 1.3E-05 | 5.0E-06 | 2.4E-05 | 1.5E-05 |
| A2B0 | A**F**D**AH**ARLM | | 3.9E-06 | 1.8E-05 | 3.0E-06 | 3.1E-05 | 1.3E-05 |
| A3B0 | A**Y**D**EH**ARLM | | 8.1E-07 | 2.3E-06 | 5.7E-07 | 3.0E-06 | 1.3E-06 |
| A4B0 | A**Y**D**VH**ARLM | | 1.7E-06 | 6.6E-06 | 1.4E-06 | 1.3E-05 | 4.1E-06 |
| A5B0 | A**Y**DQDARLM | | 3.2E-06 | 9.0E-06 | 3.0E-06 | 3.5E-05 | 1.2E-05 |
| A6B0 | A**Y**D**EN**ARLM | | 3.2E-06 | 8.3E-06 | 2.4E-06 | 2.7E-05 | 8.2E-06 |
| A7B0 | A**Y**D**VA**ARLM | | 9.3E-06 | 2.4E-05 | 8.3E-06 | 8.5E-05 | 2.3E-05 |
| A10B0 | **VY**DQ**N**ARLM | | 6.5E-06 | 2.2E-05 | 5.6E-06 | 5.8E-05 | 1.1E-05 |
| A11B0 | A**Y**DQ**W**ARLM | | 3.9E-07 | 1.1E-06 | 7.6E-08 | 8.8E-07 | 1.6E-07 |
| A0B1 | | ASSLG**AN**ELF | 1.7E-06 | 1.8E-05 | 1.7E-06 | 2.2E-06 | 5.0E-06 |
| A0B2 | | ASS**H**G**AN**ELF | 4.3E-06 | 1.6E-05 | 1.7E-06 | 1.0E-05 | 6.1E-06 |
| A0B3 | | ASSLG**SN**ELF | 2.1E-06 | 2.1E-05 | 5.1E-07 | 2.2E-05 | 5.2E-06 |
| A0B4 | | ASS**R**G**SN**ELF | 4.9E-06 | 7.6E-05 | 6.4E-06 | 5.1E-06 | 1.2E-05 |

### Example 8 Preparation of lentivirus and infection of CD8⁺ T cells

(a) Lentivirus package of wild-type and mutant NY-ESO-1 TCR. The lentivirus containing genes encoding the desired TCRs were packaged using a third generation lentivirus packaging system (Invitrogen,pLenti6/V5 Directional TOPO^{™} Cloning Kit, Cat No. K495510). In order to reduce the mispairing with endogenous TCRs of CD8⁺ T cells, the Coiled-coil structure formed by v-Fos/v-Jun was introduced to facilitate the pairing and folding of TCR α chain and β chain by referencing to previous relevant literatures and work (PNAS, 1994, 91, 11408; Mol. Ther. Oncolytics, 2017, 5, 105). The self-hydrolytic sequence of P2A *(*Nat. Biotech. 2004, 22, 589; Gene Ther., 2008, 15, 1411; J Immunother., 2008, 31, 830;) was also introduced to promote the simultaneous expression and folding of the exogenously transduced αβ TCR polycistron. The C terminus of TCR α chain and v-Jun related sequence were connected to form TCR α chain+v-Jun related sequence, referring as TRAJun. Similarly, TCR β chain+v-Fos related sequence were connected, referring as TRBFos.
v-Jun related sequence:
   SGSGRIARLEEKVKTLKAQNSELASTANMLREQVAQLKQKVMNY
v-Fos related sequence:
   SGSGLTDTLQAETDQLEDKKSALQTEIANLLKEKEKLEFILAAY
P2A related sequence: SGRAKRSGSGATNFSLLKQAGDVEENPGP

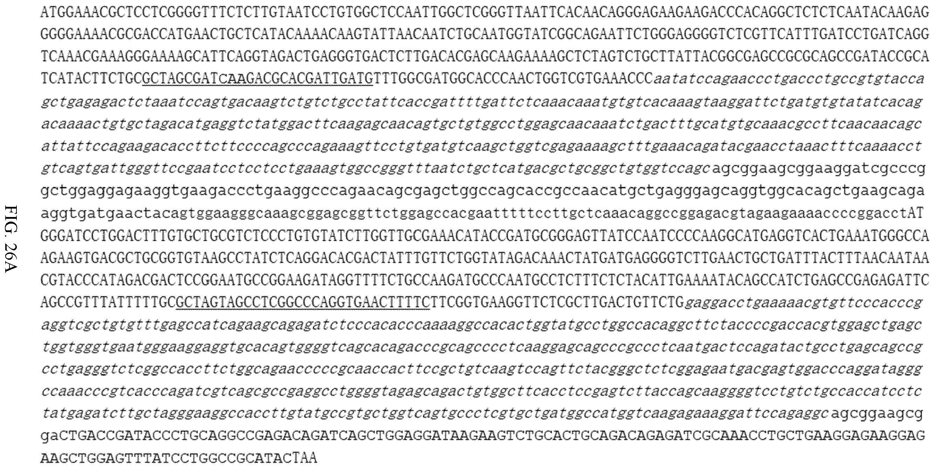

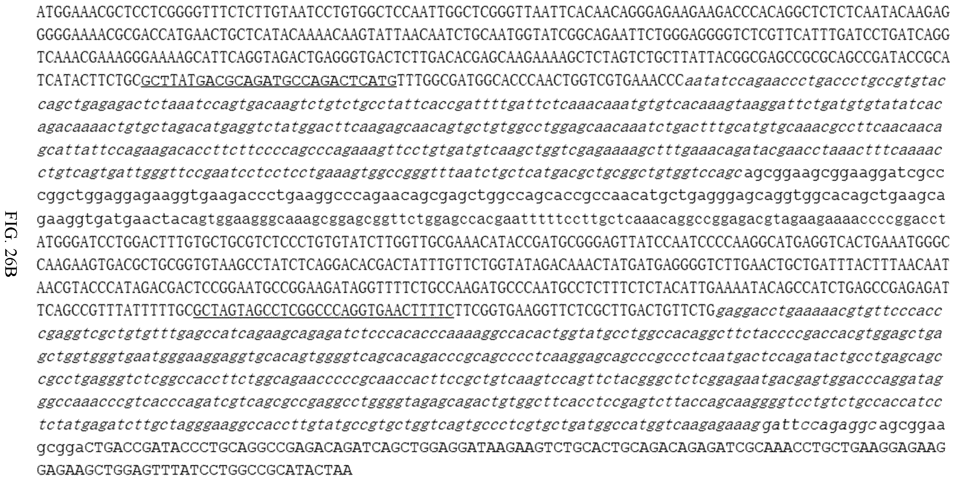

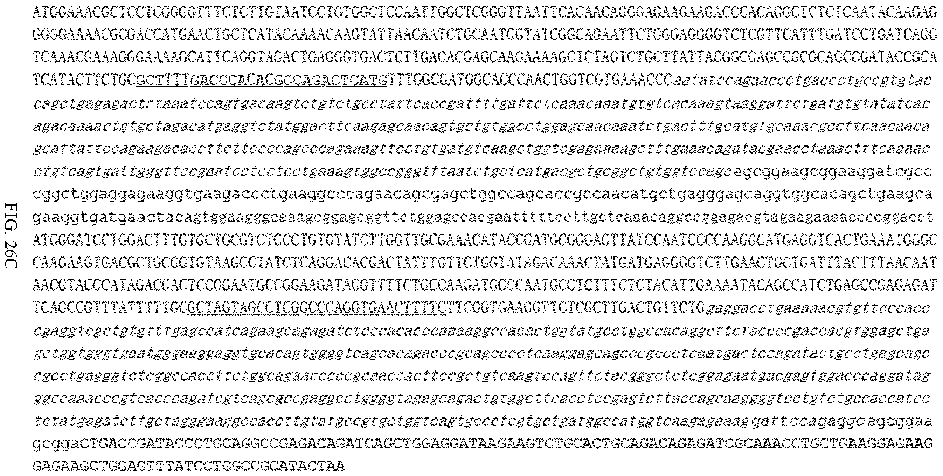

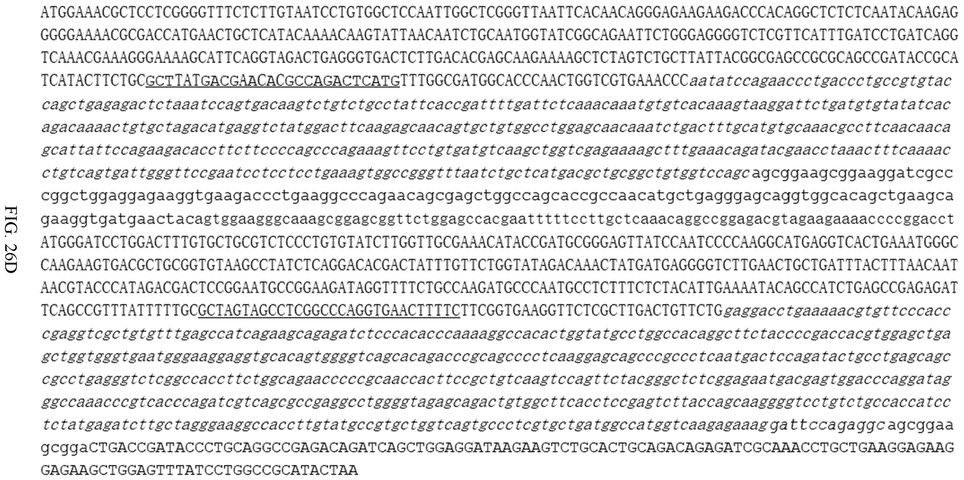

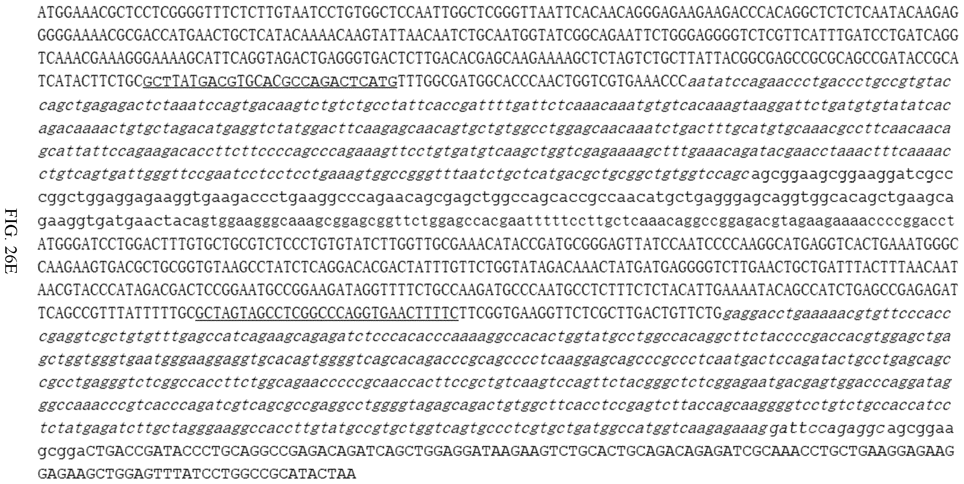

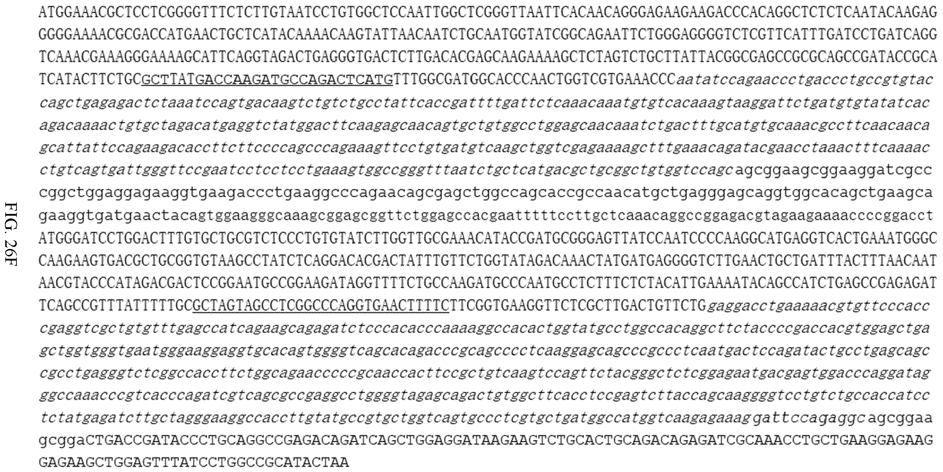

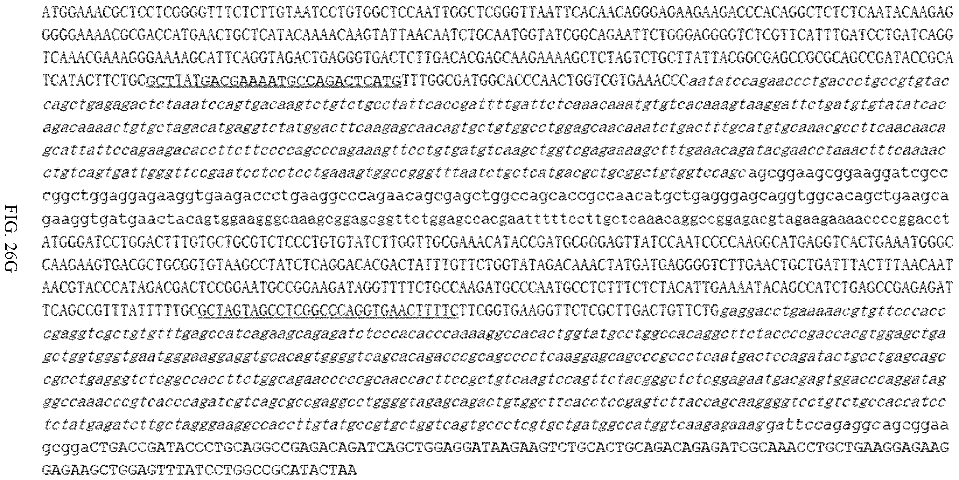

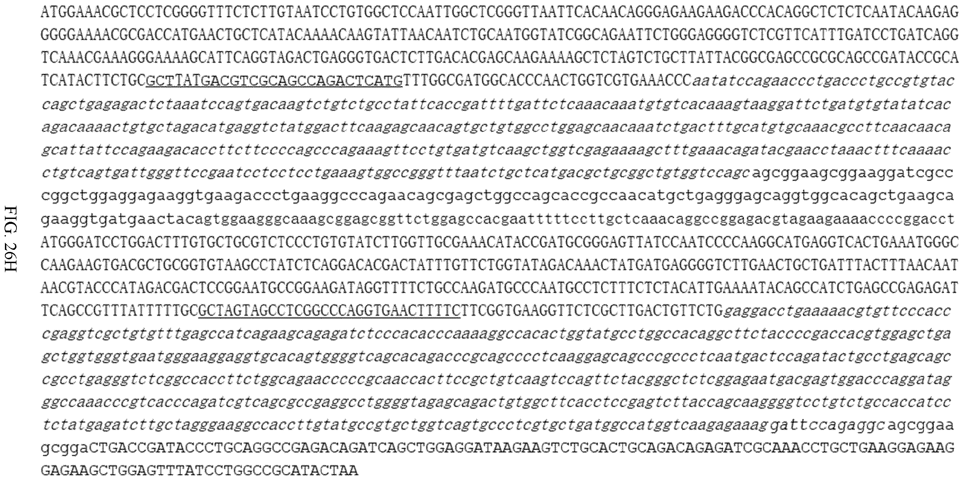

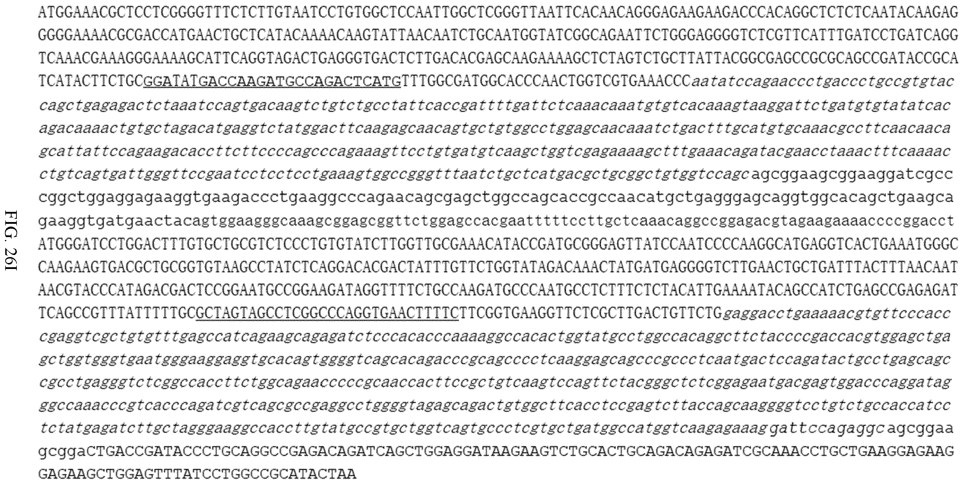

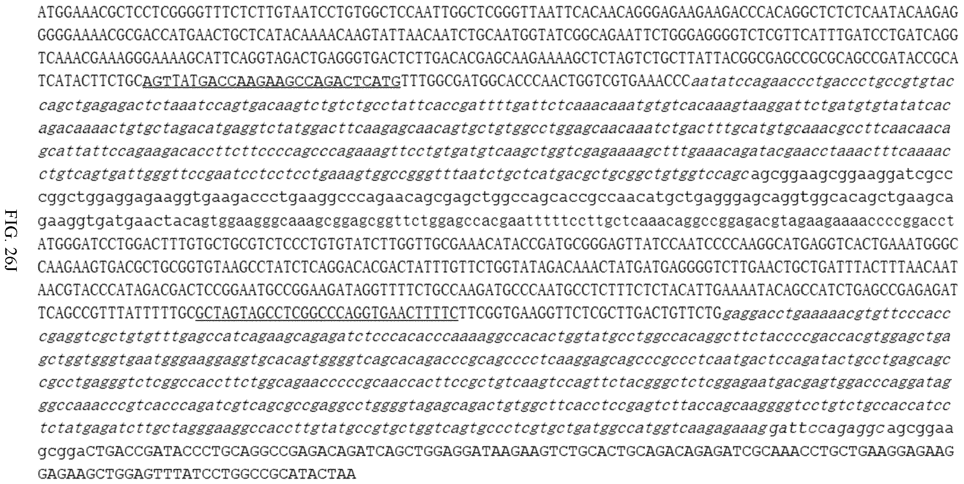

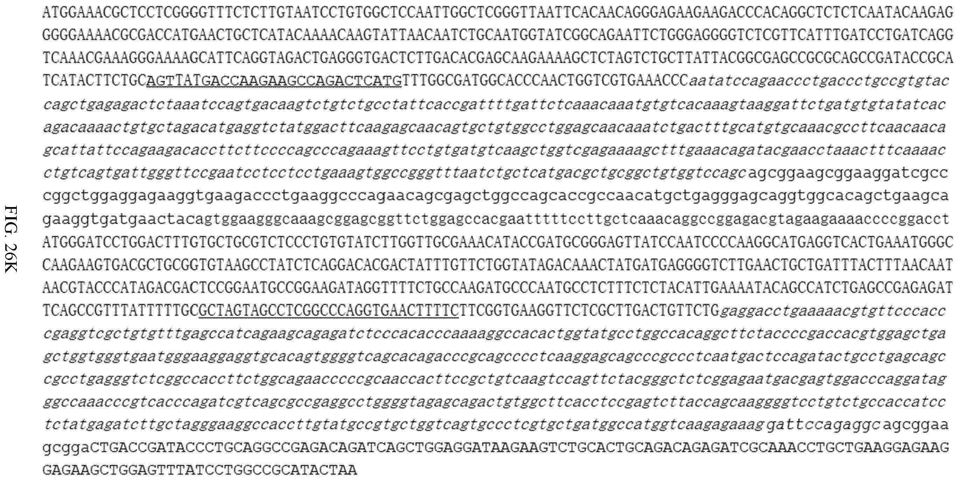

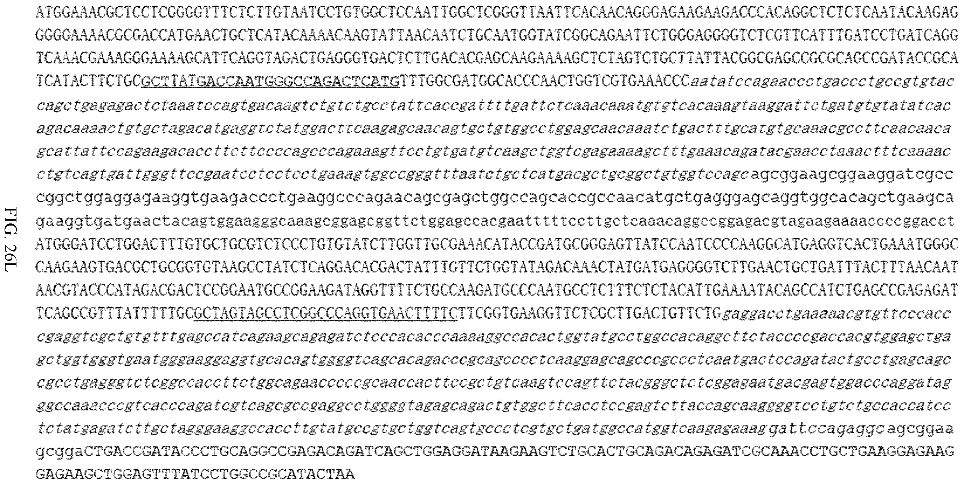

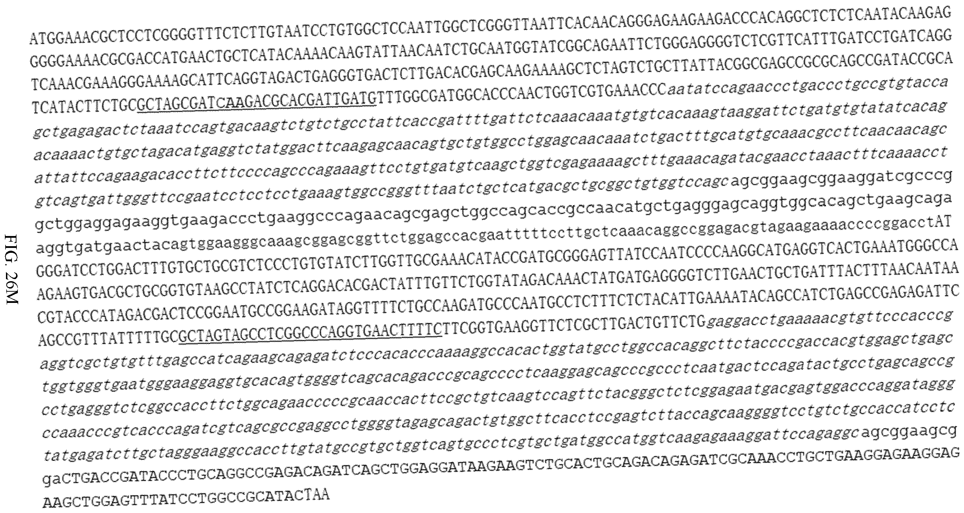

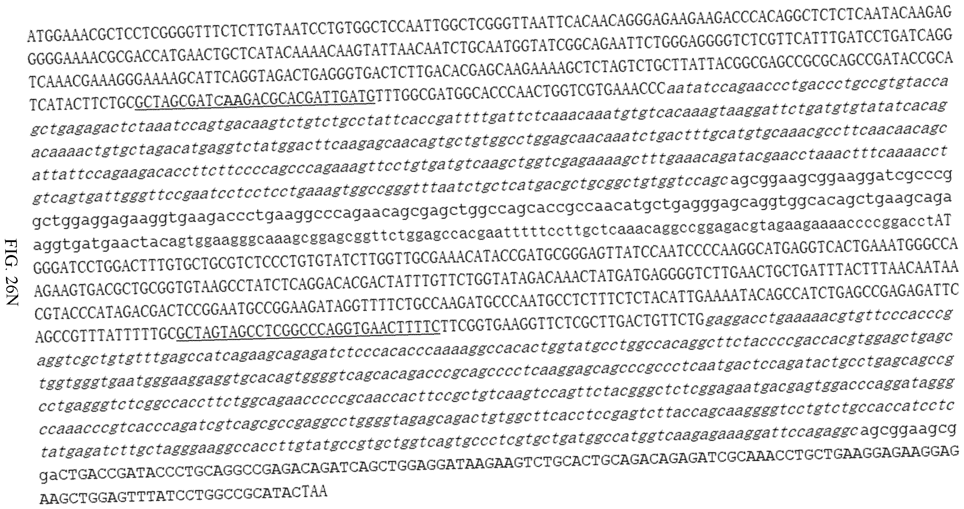

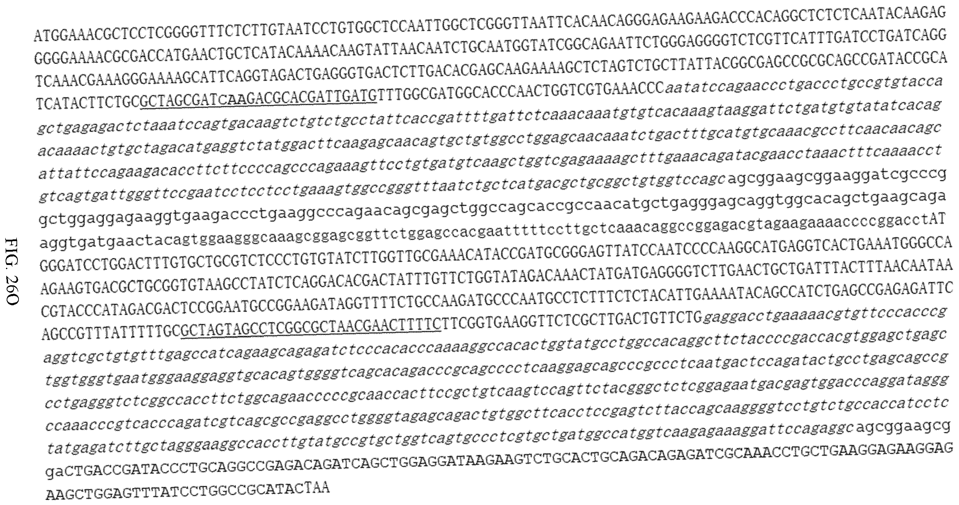

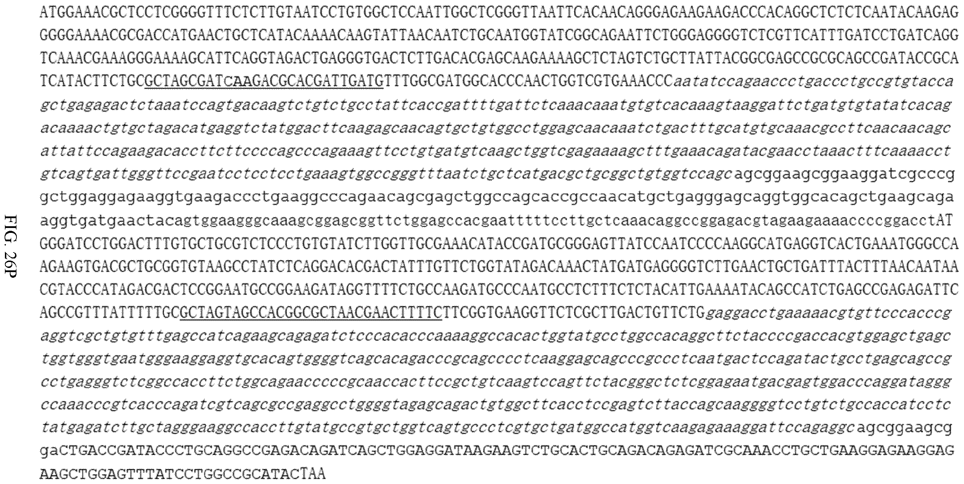

Here, the sequences of v-Jun, v-Fos and P2A were highlighted by underlines, respectively. SG and SGSG are linker sequences, RAKR is the Furin cleavage site (J Biol. Chem. 1999, 274, 23229). For example, after inserting the related sequences containing v-Fos/v-Jun and P2A, the full-length amino acid sequence of A1B0 to be expressed is: SEQ ID NO: 19+v-Jun related sequence+P2A related sequence+SEQ ID NO: 42+v-Fos related sequence, the sequence of lentiviral vector expressing A1B0 is detailed in FIG. 26B; after inserting the related sequences containing c-Fos/c-Jun and P2A, the full-length amino acid sequence of A1B0 to be expressed is: SEQ ID NO: 18+v-Jun related sequence+P2A related sequence+SEQ ID NO: 43+v-Fos related sequence, the sequence of lentiviral vector expressing A0B1 is detailed in FIG. 26 M; the sequences of lentiviral vector expressing A0B0, A1B0, A2B0, A3B0, A4B0 A5B0, A6B0, A7B0, A8B0, A9B0, A10B0, A11B0, AOB1, A0B2, A0B3, and A0B4, are detailed in FIG. 26A-FIG. 26P, respectively.

Specifically, the pseudoviruses of pLenti6-NY-ESO-1 TRAJun-2A-TRBFos for wild-type and mutant and pLenti6-eGFP, were mixed with packaging plasmids pMDLg/pRRE (addgene, Product No. k12251), pRSV-REV (addgene, Product No. 12253) and pMD2 G (addgene, Product No. 12259) at a ratio of 3:2:2:1 (refer to the product manual for details), which was transiently transfected into the 293T cells (purchased from ATCC, Cat No. CRL-3216) in logarithmic growth phase. The ratio of transfection reagent PEI-MAX (purchased from Polyscience, Cat No. 23966-1) to plasmids was 2:1 (volume-to-mass ratio), and specific operation procedures were performed according to the instructions.

On Day 3 and Day 4, the medium supernatant containing the packaged lentivirus was collected and concentrated. The collected medium supernatant was concentrated using a 50 kD molecular weight cut-off concentration tube (Merck Millipore) to a final volume of 1 ml, then aliquoted and froze at -80°C for storage. The viral titer of pseudovirus samples were determined according to the instructions of p24 ELISA kit (Clontech, Cat No. 632200). The pseudovirus of pLenti6-eGFP was also packaged as the control.

### (b) Transduction of primary CD8⁺ T cells with lentivirus containing specific NY-ESO-1 T cell receptor gene

The isolation, stimulation and proliferation of CD8⁺ T cells and usage of reagents were performed according to the references (J Immunol Methods. 2006, 310, 40; J Transl. Med. 2010, 8, 104; Nat Protoc. 2014, 9, 950). CD8+ T cells were enriched by negative separation from the blood of healthy volunteers (antibody-coupled magnetic beads were purchased from Miltenyi Biotec), and the antibody-magnetic beads were used according to the product instructions, with isolation effect of CD8⁺ T cells achieved over 90%. CD8⁺ T cells and prewashed anti-CD3/CD28 antibody-coated beads (Life Technologies, Product No. 11452D) were co-incubated and stimulated overnight in RPMI-1640 complete medium (10% FBS) containing 50 IU/mL IL-2 (Peprotech, Product No. AF-200-02) and 10 ng/mL IL-7 (Peprotech, Product No. AF-200-07), wherein the ratio of cells:beads = 1: 1.

According to the viral titer, the concentrated lentivirus was added at a ratio of MOI=10, and centrifuged 1 hour at 32°C, 900 g for infection. The lentiviral infection solution was then removed. Referencing to literatures and relevant work *(*J Immunol Methods, 1990, 128, 189), the cells were resuspended with RPMI-1640 complete medium containing 50 IU/mL IL-2 and 10 ng/mL IL-7 and cultured under the condition of 37°C/5% CO₂. Cell infection efficiency was analyzed by flow cytometry on Day 3 after the transduction, and functional tests were started on Day 5 after the transduction (e.g., IFN-γ release ELISPOT and non-radioactive cytotoxicity assays).

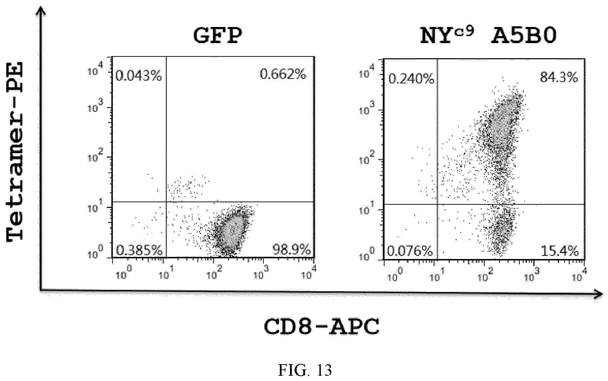

The TCR transduction efficiency in CD8⁺ T cells was analyzed by flow cytometry (PE-labeled TCR, APC-labeled CD8), and the results are detailed in FIG. 13, specific operation was referenced to the protocol of the article (Blood, 2010, 115, 3718).

### Example 9 Validation of NY-ESO-1 specific TCR function- Detection on INF-γ release of peptide loaded T₂ cells by ELISPOT

IFN-γ yield, regarded as the marker of T cell activation, was used to detect the specific activation responses of TCR-transduced T cells to target cells in this experiment, which was referenced to the literature *(*PNAS, 2011, 108, 2991).

T₂ cells were performed as the target cells in this experiment, while the effector cells were NY-ESO-1 TCR expressed CD8⁺ T cells which has been analyzed by flow cytometry in Example 8, with CD8⁺ T cells from the same volunteer used as negative control of effector cells. CD8⁺ T cells were resuspended in experimental medium (RPMI 1640 containing 10% FBS) at 2 times the final concentration needed.

PVDF ELISPOT 96-well plate (Merck Millipore, Cat No. MSIPS4510) was prepared according to the manufacturer's instructions. The anti-human IFN-γ capture antibody (human IFN-γ ELISPOT PVDF-enzyme kit, BD Company, Cat No. 551849) was diluted with sterile PBS at a ratio of 1:200 and incubated at 4°C for overnight. After the removal of excessive capture antibodies by washing, the plate was blocked with PBS containing 10% FBS for 2 hours at room temperature.

Then each experimental component was added sequentially to ELISPOT plate: 1. 10,000 T₂ cells/well; 2. 1,000 NY-ESO-1 TCR CD8⁺ double positive T cells, or negative control CD8⁺ T cells; 3. 20 µL SLLMWITQC short peptide (positive peptide) at a concentration of 10 µM, three non-specific short peptide solutions VLDGLDVLL (negative peptide 1), GLYDGMEHL (negative peptide 2), and TIHDIILECV (negative peptide 3), at a final concentration of 1 µM. All experimental groups were performed in triplicate.

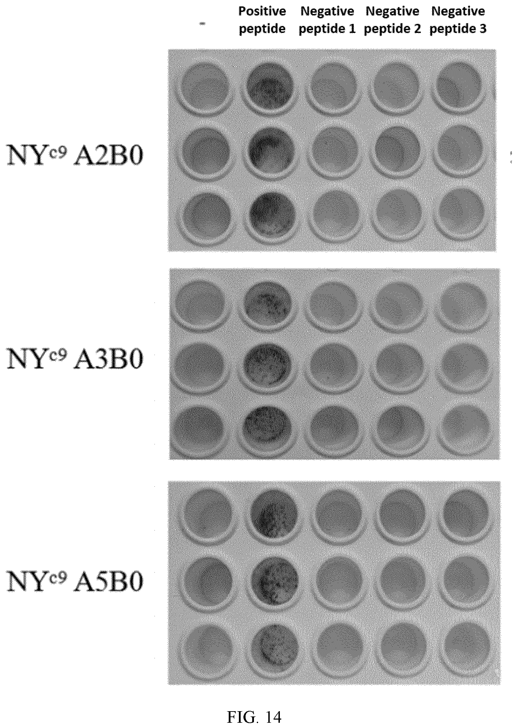

The plate was cultured overnight (37°C/5% CO₂) and then operated according to the instructions of human IFN-γ ELISPOT PVDF-enzyme kit. The medium was discarded and the plate was washed with double-distilled water and washing buffer (0.01M PBS/0.05% Tween20). The primary antibody was diluted with PBS containing 10% FBS, the plate was incubated for 2 hours at room temperature, and then washed. Again the plate was incubated for 1 hour at room temperature, with dilution of PBS containing 10% FBS. After washing with washing buffer for 3 times and PBS for 2 times, 100 µl/well of BCIP/NBT solution provided by the kit was added for color development for 5-15 min. The color reaction was terminated by removing the BCIP/NBT solution and rinsing the plate with double-distilled water. The plate was dried at room temperature until each well was completely dry. The results are shown in FIG. 14. NY^{c9} A2B0, A3B0 and A5B0 showed the signal of INF-γ release only after T₂ cells were loaded with positive peptide SLLMWITQC, while no obvious signal of INF-γ release was observed when T₂ cells were loaded without peptide, or loaded with VLDGLDVLL (negative peptide 1), GLYDGMEHL (negative peptide 2), TIHDIILECV (negative peptide 3), indicating that NY^{c9} A2B0, A3B0 and A5B0 specifically recognize HLA-A*02:01/SLLMWITQC.

### Example 10 Validation of NY-ESO-1 specific TCR function- Specific killing on tumor cell lines by LDH

This experiment is a colorimetric alternative to ⁵¹Cr release cytotoxicity assay and quantitatively determines the lactate dehydrogenase (LDH) released upon cell lysis. The experimental protocol was referenced to the literature *(*Eur. J Immunol. 1993, 23, 3217). Released LDH in culture medium was measured by the 30-min coupled enzymatic reaction, in which LDH converted a tetrazolium salt (INT) into a red methanogen (formazan). The amount of red product generated is directly proportional to the number of lysed cells. The visible absorbance data at 490 nm was collected using a standard 96-well plate reader. A375, U266, 293T and NCI-H1299 four cell lines were used as target cells in this experiment, which were inoculated 1.5×10⁴ cells per well. The effector cells (T cells) were NY-ESO-1 specific TCR expressed CD8⁺ T cells which has been analyzed by flow cytometry in Example 8. The ratios of effector cells to target cells were 10: 1/5: 1/2.5: 1/1.25: 1/0.625: 1. Homologous CD8⁺ T cells and target cells were set as the control group (5:1).

Each experimental component was added sequentially to 96-well round bottom culture microplate: 1. 100 µL target cells (prepared as described above, 1.5×10⁴ target cells/well); 2. 100 µL effector cells (prepared as described above). Addition of control groups: 1. effector cells spontaneous release: 100 µL effector cells only. 2. Target cell spontaneous release: 100 µL target cells only. 3. Target cell maximal release: 100 µL target cells only (additional lysis solution is required to be added during experiment). 4. Medium control: 200 µL medium only. 5. Volume correction wells: 200 µL medium only. All experimental groups were set in triplicate, with the final volume of 200 µL (insufficient volume was supplemented with culture medium).

Experiments were performed using CytoTox 96^{®} non-radioactive cytotoxicity assay kit (Promega, G1780, containing substrate mixture, assay buffer, lysis solution and termination buffer). Before collecting the supernatant of all wells, 20 µL lysis solution was added to target cell maximal release control wells and volume correction wells. The plate was placed at 37°C for 30 min for complete lysis of target cells.

After the mixed cells were incubated at 37°C for 24 hours, the plate was centrifuged at 250 g for 4 min. 50 µL supernatant from each well of the experimental plate was transferred to the corresponding wells of 96-well Maxisorb immunoplate. 50 µL substrate mixture was added to each well and incubated for 30 min at room temperature in dark. The reaction was terminated by adding 50 µL termination solution to each well on the plate. The absorbance value at 490 nm was read within 1 hour after the addition of termination solution.

Calculation of results: subtract the absorbance value for the medium background from all absorbance values for experimental group, target cell spontaneous release group and effector cell spontaneous release group. The corrected values obtained above were brought into the following formula to calculate percentage cytotoxicity for each effector:target ratio. Cytotoxicity (%) = 100 × (experimental - effector cell spontaneous release - target cell spontaneous release)/(target cell maximum - target cell spontaneous release).

The results showed that CD8⁺ T cells expressing TCR mutants A3B0 and A5B0, selectively killed HLA-A*02:01 and NY-ESO-1 double-positive human melanoma cells A375, as well as human myeloid malignant leukemia cells U266B1 and IM9, but did not show specific killing effects on single-positive human renal epithelial cells 293T and human lung cancer cells NCI-H1299, which is similar to that of GFP T cells for negative control.

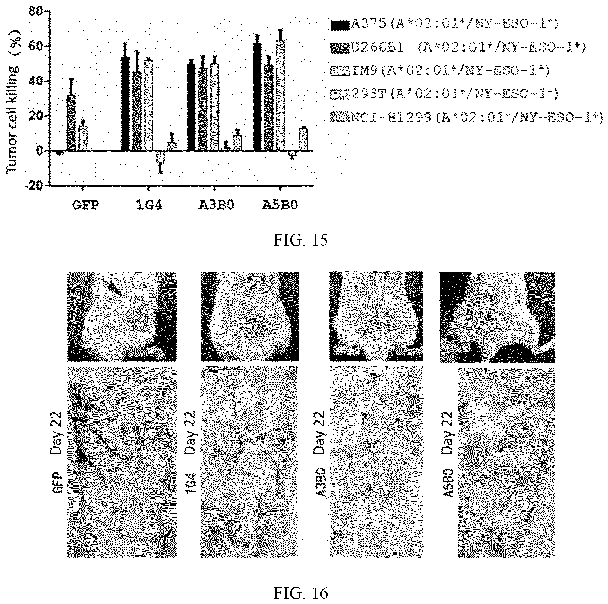

Based on the above experiments, the LDH killing assay was optimized for A3B0 and A5B0 (other conditions remained the same, with E:T optimized to 2.5:1), and a positive cell line IM9 was introduced, the experimental results are shown in FIG. 15. Based on the LDH results, the specific killing activity of A3B0 and A5B0 on tumor cells was comparable to the positive control 1G4; specifically, A3B0 and A5B0 were better than the positive control 1G4, especially the killing effects of A5B0 on A375 and IM9, which were about 10% higher.

### Example 11 Validation of NY-ESO-1 specific TCR function- xenograft assay of melanoma A375

NOD/SCID mice, female, 4 weeks old, were used for the experiments. After purchase, the animals were housed in animal feeding centers of SPF level with 5 animals per cage. The temperature of animal housing room was maintained at 20±2°C, the air was changed 15 times per hour, the daily lighting time was equally divided (12 hours of light: 12 hours of darkness), and the relative humidity was maintained at 50%-55%. The cages were embedded with hardwood chips, each group of mice were uniformly fed with standard diet and clean drinking water. Mice in each group were acclimatized for 1 week before being subject to subsequent experiments.

The mice were randomly divided into negative control group (GFP-T cells), positive control group (1G4 TCR-T cells; wherein 1G4 is the α95-LY mutant mentioned in J. Immunol. 2008, 180, 6116. and its sequence was synthesized by a biological company), and experimental groups (A3B0 TCR-T cells and A5B0 TCR-T cells), with 5 mice in each group and 20 mice in total. The mice were ear punched for incision marking. Tumor animal model was established via subcutaneous inoculation route of cells, specifically, each mouse was shaved at the back of right hind limb and inoculated with a mixture of 5×10⁶ A375 cells (human melanoma cells) and 1.5×10⁷ T cells (cells were collected in PBS buffer). The total volume of inoculated cells was 200 µL per mouse. After cell inoculation, 100 µL human recombinant IL-2 (concentration of storage solution was 500,000 units/mL) was injected intraperitoneally into each mouse for 5 consecutive days. Ten days after inoculation, tumor volume measurement was started, which was measured every two days thereafter, with long and short diameters recorded to calculate volume size for tumor growth curve plotting. The formula to calculate tumor volume is V=1/6×π×a×b² (a is the long diameter and b is the short diameter). The mice were euthanized by CO₂ after one month.

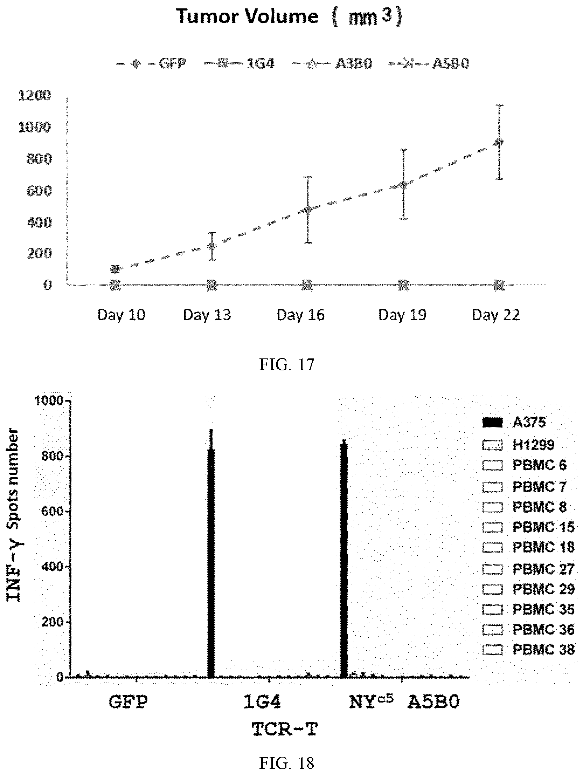

The growth status of tumor-bearing mice in each group is shown in FIG. 16. Compared with negative control group GFP-T cells, 1G4 TCR-T cells, A3B0 TCR-T cells and A5B0 TCR-T cells were all able to effectively kill the tumor cells A375 and inhibit the growth of tumors with no significant change of tumor growth curves over time, as detailed in FIG. 17 (given that the effects of A3B0 TCR-T cells were comparable to A5B0 TCR-T cells, A5B0 was used as an example in the following examples for subsequent experiments).

### Example 12 Validation of NY-ESO-1 specific TCR function- Specific INF-γ release of PBMC from healthy individuals

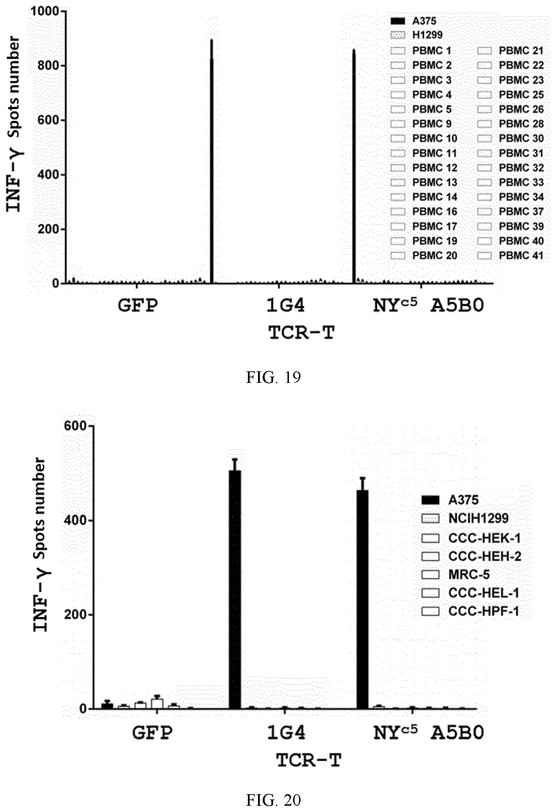

In order to investigate the safety of NY^{C9} high-affinity mutants, PBMC from 40 healthy individuals were performed for safety investigation by the INF-γ release assay used in Example 9. PBMC of 10 healthy individuals contained HLA-A*02:01 typing, while PBMC from 30 healthy individuals did not contain the HLA-A*02:01 typing. According to the results of INF-γ release, NY^{c9} A5B0 TCR-T cells did not show significant reaction with PBMC from 40 healthy individuals, regardless of whether the HLA typing of 10 PBMC is A*02:01 (according to INF-γ release data of NY^{c9} A5B0 on PBMC from 10 healthy individuals with HLA-A*02:01 typing, its response intensity was comparable with negative and positive controls; as detailed in FIG. 18), or the 30 PBMC is non-HLA-A*02:01 typing (according to INF-γ release data of NY^{c9} A5B0 on PBMCs from 30 healthy individuals with non-HLA-A*02:01 typing, its response intensity is comparable to negative and positive controls; as detailed in FIG. 19).

### Example 13 Validation of NY-ESO-1 specific TCR function- Specific INF-γ release of primary cells from healthy individuals

In order to investigate the safety of NY^{C9} high-affinity mutants, primary cells from 5 important organs were performed for safety investigation by the INF-γ release assay used in Example 9. Here, CCC-HEK-1, MRC-5, CCC-HEH-2, CCC-HEL-1, and CCC-HPF-1 were primary cells originated from embryos, and purchased from National Experimental Cell Resource Sharing Platform, while MRC-5 was from ATCC. According to the results of INF-γ release, the response intensity of NY^{c9} A5B0 TCR-T cells is comparable to that of negative and positive controls, with no significant reaction with primary cells from these important organs (details are shown in FIG. 20).

### Example 14 Validation of NY-ESO-1 specific TCR function- Functional verification of multiple HLA-A*02:0x typing

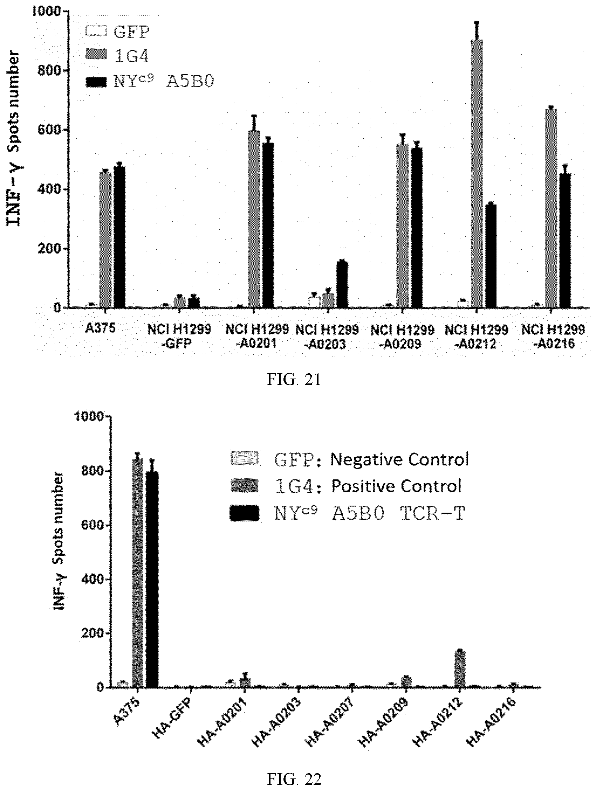

In order to investigate the compatibility of NY^{C9} high-affinity mutants with multiple HLA-A*02:0x typing, NCI-H1299 tumor cell line which is HLA-A*02 negative and NY-ESO-1 positive, were infected by lentivirus carrying the HLA-A02:0x gene. The INF-γ release assay used in Example 9, was performed to analyze the INF-γ release on NCI-H1299 transduced with different HLA-A*02:0x, for evaluation of the activity on high-affinity mutant NY^{c9} A5B0 against multiple HLA-A*02:0x typing. FIG. 21 shows that NY^{c9} A5B0 exhibited apparent activity against HLA-A*02:03, better activity against HLA-A*02:12, and comparable activity against HLA-A*02:01, HLA-A*02:09, and HLA-A*02:16.

### Example 15 Function of NY-ESO-1 specific TCR- Safety validation of multiple HLA-A*02:0x typing

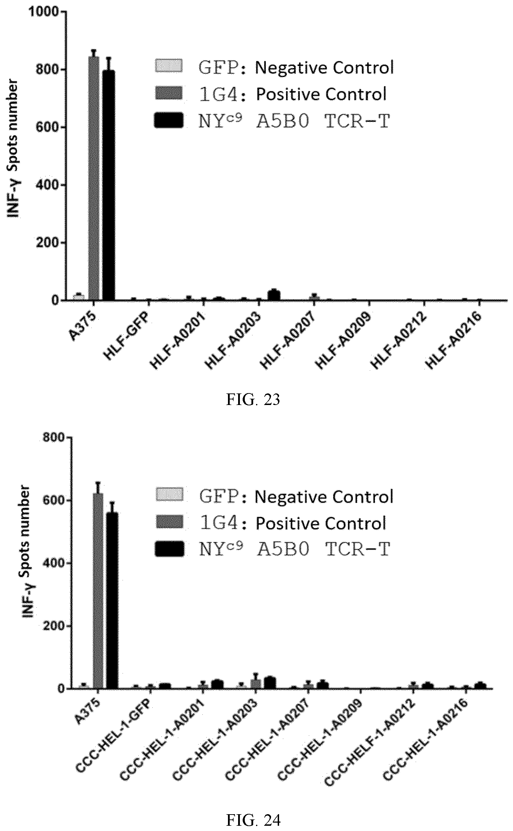

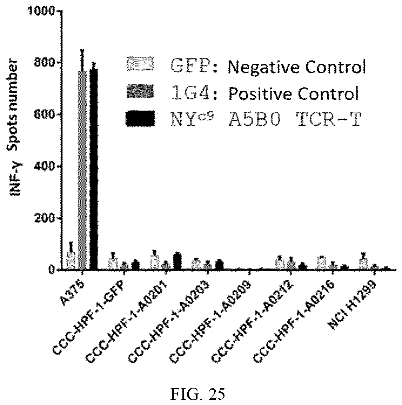

In order to investigate the safety of high-affinity mutant NY^{c9} A5B0 against multiple HLA-A*02:0x typing, various human primary cells such as human astrocytes (HA), human lung fibroblasts (HLF), human embryonic liver cells (CCC-HEL) and human embryonic pulmonary fibroblasts (CCC-HPF), were infected via lentivirus carrying the HLA-A02:0x gene. The INF-γ release assay used in Example 9, was then performed to analyze the INF-γ release on human primary cells transduced with different HLA-A*02:0x, to evaluate the safety of high-affinity mutant NY^{c9} A5B0 against multiple HLA-A*02:0x typing. FIG. 22, FIG. 23, FIG. 24, and FIG. 25 respectively show the results of INF-γ release on NY^{c9} A5B0 against human astrocytes (HA), human lung fibroblasts (HLF), human embryonic liver cells (CCC-HEL) and human embryonic pulmonary fibroblasts (CCC-HPF) which are infected by multiple HLA-A*02:0x typing, indicating the recognition of NY^{c9} A5B0 on multiple HLA-A*02:0x typing is safe.

Although the specific embodiments of the present invention are described above, it should be understood by those skilled in the art that these are merely illustrative examples and that a variety of changes or modifications to these embodiments can be made without departing from the principles and substance of the present invention. Therefore, the scope of protection of the present invention is limited by the appended claims.

## Claims

1. A TCR comprising a TCR α chain variable region and/or a TCRβ chain variable region, wherein the TCR can specifically recognize and bind to HLA-A*02:01/SLLMWITQC, the affinity *K_{D1}* of the TCR to the HLA-A*02:01/SLLMWITQC is 0.1-10 µM, preferably 0.39-9.3 µM, more preferably 0.81-3.2 µM, wherein, the TCR can also specifically recognize and bind to one or more of HLA-A*02:03/SLLMWITQC, HLA-A*02:09/SLLMWITQC, HLA-A*02:12/SLLMWITQC, and HLA-A*02:16/SLLMWITQC, and the affinity *K_{D2}* is 0.1-85 µM, preferably 0.57-27 µM, more preferably 1.3-10 µM;
wherein: the affinity *K_{D}* of the TCR to the HLA-A*02:16/SLLMWITQC is preferably 0.16-23 µM, more preferably 1.3-12 µM;
the affinity *K_{D}* of the TCR to the HLA-A*02:03/SLLMWITQC is preferably 1.1-76 µM, more preferably 2.3-9 µM;
the affinity *K_{D}* of the TCR to the HLA-A*02:09/SLLMWITQC is preferably 0.1-8.3 µM, more preferably 0.51-5.6 µM; further more preferably 0.57-3 µM;
the affinity *K_{D}* of the TCR to the HLA-A*02:12/SLLMWITQC is preferably 0.88-85 µM, more preferably 3-35 µM; further more preferably 5.1-10 µM.

2. The TCR according to claim 1, wherein, the TCR can specifically recognize and bind to the HLA-A*02:09/SLLMWITQC and/or the HLA-A*02:12/SLLMWITQC;
or, the TCR can specifically recognize and bind to the HLA-A*02:03/SLLMWITQC and/or the HLA-A*02:16/SLLMWITQC.

3. The TCR according to claim 2, wherein, the amino acid sequences of CDR1, CDR2, and CDR3 of the TCR α chain variable region are as shown in SEQ ID NO: 71, SEQ ID NO: 72, and SEQ ID NO: 1 respectively, or as shown in derived sequences of SEQ ID NO: 71, SEQ ID NO: 72, and SEQ ID NO: 1 respectively; preferably:
the derived sequence of SEQ ID NO: 71 has 89% or more, 90% or more, 91% or more, 92% or more, 93% or more, 94% or more, 95% or more, 96% or more, 97% or more, 98% or more, or 99% or more identity to the sequence as shown in SEQ ID NO: 71,
the derived sequence of SEQ ID NO: 72 has 89% or more, 90% or more, 91% or more, 92% or more, 93% or more, 94% or more, 95% or more, 96% or more, 97% or more, 98% or more, or 99% or more identity to the sequence as shown in SEQ ID NO: 72,
the derived sequence of SEQ ID NO: 1 has 55% or more identity to the sequence as shown in SEQ ID NO: 1, preferably 66.67% or more, more preferably 77.78% or more, further more preferably 88.89% or more;
the derived sequence of SEQ ID NO: 1 is preferably obtained by 1, 2, 3 or 4 point mutations in the sequence as shown in SEQ ID NO: 1,
more preferably, the derived sequence of SEQ ID NO: 1 is the amino acid sequence in which at least a substitution of amino acid occurs at position 2 of the sequence as shown in SEQ ID NO: 1, the amino acid after the substitution is aromatic amino acid; preferably tyrosine or phenylalanine; the substitution on the amino acid sequence as shown in SEQ ID NO: 1 preferably further occurs at positions 1, 4 and/or 5;
further more preferably, when the amino acid at position 2 after the substitution is tyrosine, the substitution in the amino acid sequence as shown in SEQ ID NO: 1 further occurs at positions 1, 4 and/or 5; when the amino acid at position 2 after the substitution is phenylalanine, the substitution in the amino acid sequence as shown in SEQ ID NO: 1 further occurs at position 5; for position 1, the amino acid after the substitution is preferably valine; for position 4, the amino acid after the substitution is preferably glutamic acid, valine or alanine; for position 5, the amino acid after the substitution is preferably histidine, asparagine, tryptophan or alanine;
optimally, the derived sequence of SEQ ID NO: 1 is as shown in any one of SEQ ID NOs: 3-13 in sequence listing.

4. The TCR according to any one of claims 1-3, wherein, the amino acid sequences of CDR1, CDR2, and CDR3 of the TCR β chain variable region are as shown in SEQ ID NO: 73, SEQ ID NO: 74, and SEQ ID NO: 2 respectively, or as shown in derived sequences of SEQ ID NO: 73, SEQ ID NO: 74, and SEQ ID NO: 2 respectively; preferably:
the derived sequence of SEQ ID NO: 73 has 89% or more, 90% or more, 91% or more, 92% or more, 93% or more, 94% or more, 95% or more, 96% or more, 97% or more, 98% or more, or 99% or more identity to the sequence as shown in SEQ ID NO: 73,
the derived sequence of SEQ ID NO: 74 has 89% or more, 90% or more, 91% or more, 92% or more, 93% or more, 94% or more, 95% or more, 96% or more, 97% or more, 98% or more, or 99% or more identity to the sequence as shown in SEQ ID NO: 74,
the derived sequence of SEQ ID NO: 2 has 70% or more identity to the sequence as shown in SEQ ID NO: 2, preferably 80% or more, more preferably 77.78% or more, further more preferably 90% or more;
the derived sequence of SEQ ID NO: 2 is preferably obtained by 2 or 3 point mutations in the sequence as shown in SEQ ID NO: 2;
more preferably, the derived sequence of SEQ ID NO: 2 is the amino acid sequence in which at least a substitution of amino acid occurs at position 7 of the sequence as shown in SEQ ID NO: 2, amino acid after the substitution is preferably asparagine; the substitution in the amino acid sequence as shown in SEQ ID NO: 2 preferably further occurs at position 6, the amino acid after the substitution is preferably serine or alanine; the substitution in the amino acid sequence as shown in SEQ ID NO: 2 preferably further occurs at position 4, the amino acid after the substitution is preferably histidine or arginine;
further more preferably, when the amino acid at position 6 after the substitution is serine, the amino acid after the substitution for position 4 is arginine; when the amino acid at position 6 after the substitution is alanine, the amino acid after the substitution for position 4 is histidine;
optimally, the derived sequence of SEQ ID NO: 2 is as shown in any one of SEQ ID NOs: 14-17 in sequence listing.

5. The TCR according to claim 4, wherein, the amino acid sequence of CDR3 of the TCR α chain variable region is as shown in SEQ ID NO: 3, and the amino acid sequence of CDR3 of the TCR β chain variable region is as shown in SEQ ID NO: 2; the amino acid sequence of CDR3 of the TCR α chain variable region is as shown in SEQ ID NO: 4, and the amino acid sequence of CDR3 of the TCR β chain variable region is as shown in SEQ ID NO: 2; the amino acid sequence of CDR3 of the TCR α chain variable region is as shown in SEQ ID NO: 5, and the amino acid sequence of CDR3 of the TCR β chain variable region is as shown in SEQ ID NO: 2; the amino acid sequence of CDR3 of the TCR α chain variable region is as shown in SEQ ID NO: 6, and the amino acid sequence of CDR3 of the TCR β chain variable region is as shown in SEQ ID NO: 2; the amino acid sequence of CDR3 of the TCR α chain variable region is as shown in SEQ ID NO: 1, and the amino acid sequence of CDR3 of the TCR β chain variable region is as shown in SEQ ID NO: 14; the amino acid sequence of CDR3 of the TCR α chain variable region is as shown in SEQ ID NO: 1, and the amino acid sequence of CDR3 of the TCR β chain variable region is as shown in SEQ ID NO: 15; the amino acid sequence of CDR3 of the TCR α chain variable region is as shown in SEQ ID NO: 1, and the amino acid sequence of CDR3 of the TCR β chain variable region is as shown in SEQ ID NO: 16; the amino acid sequence of CDR3 of the TCR α chain variable region is as shown in SEQ ID NO: 1, and the amino acid sequence of CDR3 of the TCR β chain variable region is as shown in SEQ ID NO: 17; the amino acid sequence of CDR3 of the TCR α chain variable region is as shown in SEQ ID NO: 7, and the amino acid sequence of CDR3 of the TCR β chain variable region is as shown in SEQ ID NO: 2; the amino acid sequence of CDR3 of the TCR α chain variable region is as shown in SEQ ID NO: 8, and the amino acid sequence of CDR3 of the TCR β chain variable region is as shown in SEQ ID NO: 2; the amino acid sequence of CDR3 of the TCR α chain variable region is as shown in SEQ ID NO: 9, and the amino acid sequence of CDR3 of the TCR β chain variable region is as shown in SEQ ID NO: 2; the amino acid sequence of CDR3 of the TCR α chain variable region is as shown in SEQ ID NO: 12, and the amino acid sequence of CDR3 of the TCR β chain variable region is as shown in SEQ ID NO: 2; or, the amino acid sequence of CDR3 of the TCR α chain variable region is as shown in SEQ ID NO: 13, and the amino acid sequence of CDR3 of the TCR β chain variable region is as shown in SEQ ID NO: 2.

6. The TCR according to any one of claims 1-5, wherein, the TCR α chain variable region or the TCR β chain variable region further comprises one or more of FR1, FR2, FR3, and FR4;
preferably, the FR1, the FR2, and the FR3 in the TCR α chain variable region are originated from germline TRAV17 or a mutant thereof, and/or the FR4 is originated from germline TRAJ-31 or a mutant thereof;
and/or, the FR1, the FR2, and the FR3 in the TCR β chain variable region are originated from germline TRBV12-4 or a mutant thereof, and/or the FR4 is originated from germline TRBJ2-2 or a mutant thereof;
more preferably:
when the FR1, the FR2, and the FR3 in the TCR α chain variable region are originated from germline TRAV17 and the FR4 is originated from germline TRAJ-31, the amino acid sequence of the TCR α chain variable region is as shown in any one of SEQ ID NOs: 18-29; when the FR1, the FR2, and the FR3 in the TCR β chain variable region are originated from the germline TRBV12-4 and the FR4 is originated from the germline TRBJ2-2, the amino acid sequence of the TCR β chain variable region is as shown in any one of SEQ ID NOs: 42-46;
or, when the FR1, the FR2, and the FR3 in the TCR α chain variable region are originated from the mutant of germline TRAV17 and the FR4 is originated from the mutant of germline TRAJ-31, the amino acid sequence of the TCR α chain variable region is as shown in any one of SEQ ID NOs: 30-41; when the FR1, the FR2, and the FR3 in the TCR β chain variable region are originated from the mutant of germline TRBV12-4 and the FR4 is originated from the mutant of germline TRBJ2-2, the amino acid sequence of the TCR β chain variable region is as shown in any one of SEQ ID NOs: 47-51; the TCR preferably is an ScTCR, wherein the TCR α chain variable region and the TCR β chain variable region in the ScTCR are connected by a linker.

7. The TCR according to any one of claims 1-6, wherein, the TCR α chain and/or the TCR β chain of the TCR further comprises a constant region, the constant region of the TCR α chain is preferably originated from germline TRAC; and/or the constant region of the TCR β chain is preferably originated from germline TRBC2;
preferably, the TCR α chain and/or the TCR β chain of the TCR further comprises an extra-membrane region and a transmembrane region; more preferably, the TCR α chain and/or the TCR β chain of the TCR further comprises an intracellular sequence.

8. A nucleic acid encoding the TCR according to any one of claims 1-7.

9. A vector comprising the nucleic acid according to claim 8, the vector is preferably a lentiviral vector; the nucleic acid encodes the TCR α chain and the TCR β chain in a single open reading frame, or in two different open reading frames, respectively.

10. A cell comprising the nucleic acid according to claim 8 or the vector according to claim 9; preferably, the cell is a T cell or a stem cell, the T cell is preferably a CD8⁺ T cell.

11. An isolated or non-naturally occurred cell presenting the TCR according to any one of claims 1-7, the cell is preferably a T cell.

12. A pharmaceutical composition comprising the TCR according to any one of claims 1-7 or the cell according to claim 10; preferably, the pharmaceutical composition further comprises a pharmaceutically acceptable carrier.

13. A kit comprising the TCR according to any one of claims 1-7, the nucleic acid according to claim 8, the cell according to claim 10 or 11, or the pharmaceutical composition according to claim 12.

14. A use of the TCR according to any one of claims 1-7, the cell according to claim 10 or 11, or the pharmaceutical composition according to claim 10 in the manufacture of a medicament for preventing and/or treating a NY-ESO-1 expression-associated tumor; preferably, the tumor comprises synovial sarcoma, liposarcoma, myeloid malignant leukemia, malignant melanoma, ovarian cancer, neuroblastoma, prostate cancer, bladder cancer, breast cancer, hepatocellular carcinoma, non-small cell lung cancer, oral squamous carcinoma, and esophageal cancer.

15. A method of preventing and/or treating a NY-ESO-1 expression-associated tumor comprising administering the TCR according to any one of claims 1-7, the nucleic acid according to claim 8, the cell according to claim 10 or 11, or the pharmaceutical composition according to claim 10 to a subject in need thereof; preferably, the tumor comprises synovial sarcoma, liposarcoma, myeloid malignant leukemia, malignant melanoma, ovarian cancer, neuroblastoma, prostate cancer, bladder cancer, breast cancer, hepatocellular carcinoma, non-small cell lung cancer, oral squamous carcinoma, and esophageal cancer.
